# EUROPEAN PATENT APPLICATION

(11) **EP 4 266 151 A1**
(43) Date of publication of application: **25.10.2023**
(21) Application number: 23169167.6
(22) Date of filing: 21.04.2023
(51) Int. Cl.: G06F 1/16

(54) **SYSTEMS FOR OPTIMIZING POWER CONSUMPTION OF A WEARABLE DEVICE USING SENSOR-BASED DETERMINATIONS OF POSITIONAL STATES OF A PORTION OF THE WEARABLE DEVICE, AND METHODS OF USE THEREOF**

(30) Priority: 22.04.2022 US 202263334030 P; 18.04.2023 US 202318302730
(71) Applicant: Meta Platforms Technologies, LLC, Menlo Park, CA 94025 (US)
(72) Inventor: RAJAGOPAL, Nagalakshmi, Menlo Park, 94025 (US); WRIGHT, Derek William, Menlo Park, 94025 (US); APARICIO, Edwin Corona, Menlo Park, 94025 (US); TANKIEWICZ, Szymon Michal, Menlo Park, 94025 (US); SHAGA, Ravi Krishna, Menlo Park, 94025 (US); CALDERON, Ramiro, Menlo Park, 94025 (US); SRINIVASAN, Nishant, Menlo Park, 94025 (US); CHU, Shan, Menlo Park, 94025 (US); SHARMA, Priyanka, Menlo Park, 94025 (US); YIN, Lei, Menlo Park, 94025 (US); HUANG, Lidu, Menlo Park, 94025 (US)
(74) Representative: Murgitroyd & Company

(57) **Abstract**

Methods and wearable devices for optimizing power consumption using sensor-based position and use determinations are described here. One example method is performed at a device that includes a first sensor configured to operate with a first power consumption rate and a second power consumption rate. The method includes, while a component associated with the second sensor is in an inactive state, receiving first sensor data, and determining whether the first sensor data indicates movement of the device. The method also includes, when movement of the device is indicated, operating the second sensor in an active state. The method further includes, after activating the second sensor, when second sensor data from the second sensor indicates that the device has been placed on a user's body, continuing to operate the second sensor in the active state.

## Description

### RELATED APPLICATIONS

This application claims priority to U.S. Provisional Application Serial No. 63/334,030, filed April 22, 2022, which is hereby incorporated by reference, in its entirety.

### TECHNICAL FIELD

This application relates generally to wearable devices, and more particularly, to optimizing energy consumption and processor utilization (e.g., by activating and deactivating various sensors, along with determining when to activate other hardware components such as a camera) of a wearable device based on sensor-based determinations of states (e.g., positional-based and use-based states) of a portion of the wearable device (e.g., a capsule portion, including a display, of a wrist-wearable device is determined to be in various positional-based or use-based states, including states corresponding to on-wrist, off-wrist, and activity being performed, among others).

### BACKGROUND

Conventional wearable devices often have small batteries and certain power-hungry sensors that drain battery life quicker than end-users typically expect. Some wearable devices reduce the number of sensors found on the device to prolong battery life. Using such an approach, however, reduces the efficacy of the wearable device, as it is not as feature rich as it could be. Some wearable devices also, or alternatively, forgo inclusion of certain types of power-hungry sensors, which can limit the interactions and gestures available at those devices. Power-hungry hardware components other than sensors (e.g., a camera and associated image-processing circuitry and/or software) can also drain battery life quickly, so techniques to efficiently enable and disable the power-hungry hardware components (particularly for wearable devices) are needed.

Additionally, electronic devices (including wearable devices) can also include various components that are used for a single (or small number of) function(s) and are often idle for long periods of time when that single function is not needed. This leads to inefficient utilization of these various components (and can also lead to including redundant components that might be unnecessary if these various components are repurposed to serve other functions, thereby allowing for replacement of some redundant components).

### SUMMARY

The embodiments discussed herein address one or more of the problems discussed above, for example by activating and deactivating different sensors (and component subsets of sensors) based on determined states of the device (e.g., charging, off-wrist, in-hand, on-wrist, on-wrist while sleeping, on-wrist while exercising etc.,). For example, powering high-power consumption sensors such as photoplethysmography (PPG) sensors and/or neuromuscular-signal sensors (e.g., sensors for detecting signals associated with muscle activities at a user's wrist and digits, such as electromyography sensors) is only necessary when the wearable device is on the wrist of the user, and keeping the PPG and/or neuromuscular-signal sensors on (or at least partially on, such as by turning on all or a portion of hardware and/or software components associated with the sensors) when it is removed from the wrist would waste valuable power. Thus, there is a need to have a process for deactivating and activating sensors based on the needs on the wearable device. There is also a need for using the sensor-based determinations of positional and use states to determine when to enable and disable certain hardware components (e.g., a camera of a wrist-wearable device).

Turning to the problem of underutilized components that was described above, techniques are described herein to address this problem by having the underutilized components of the wearable device serve multiple purposes, which further reduces complexity and power consumption (e.g., by allowing the repurposed components to be used instead of a now-redundant component), and thereby allows for repurposing components that would otherwise remaining underutilized. For example, an NFC coil can be configured to serve its normal communication function, but can, at certain points in time such as when it is not needed for its communication function, be repurposed to operate as a sensor, such as being used to detect a capacitance of a nearby surface or object (such as capacitance from one or more digits of a user's hand). In some embodiments, the detecting of capacitance using a repurposed component can help allow the device to determine (as an alternative to, or used in addition to, other techniques described herein) whether the wearable device is on a wrist of a user, off a wrist of the user, or in the hand of the user.

Now a few of the inventive techniques described herein will each be briefly summarized. In a first aspect summarized below in the paragraphs beginning with (A1), techniques for optimizing power consumption in a wearable device with at least two sensors are described, including a technique for using data from a first of the at least two sensors (e.g., an inertial measurement unit, which is a low-power sensor) to determine when to move a second of the at least two sensors (e.g., a neuromuscular-signal sensor that is used to detect signals associated with muscle movements of a user of the wearable device) to an active state. The second of the at least two sensors can be a power-hungry sensor and thus use of this technique ensures better management of limited power and computing resources at the wearable device by ensuring that the second power-hungry sensor is only active when it needs to be active. In a second aspect summarized below in paragraphs beginning with (G1), techniques for optimizing when to use three different sensors of a wearable device to assist with determining cradle, wrist, and activity states of the wearable device are described. In a third aspect summarized below in paragraphs beginning with (L1), techniques for repurposing a metallic data-transmission component normally used in conjunction with a data-communication function are provided that allow for repurposing the metallic data-transmission component to perform a sensor function (e.g., be used for detecting capacitance of a nearby surface or object).

Each of these three aspects is summarized in turn below, starting with the first aspect.

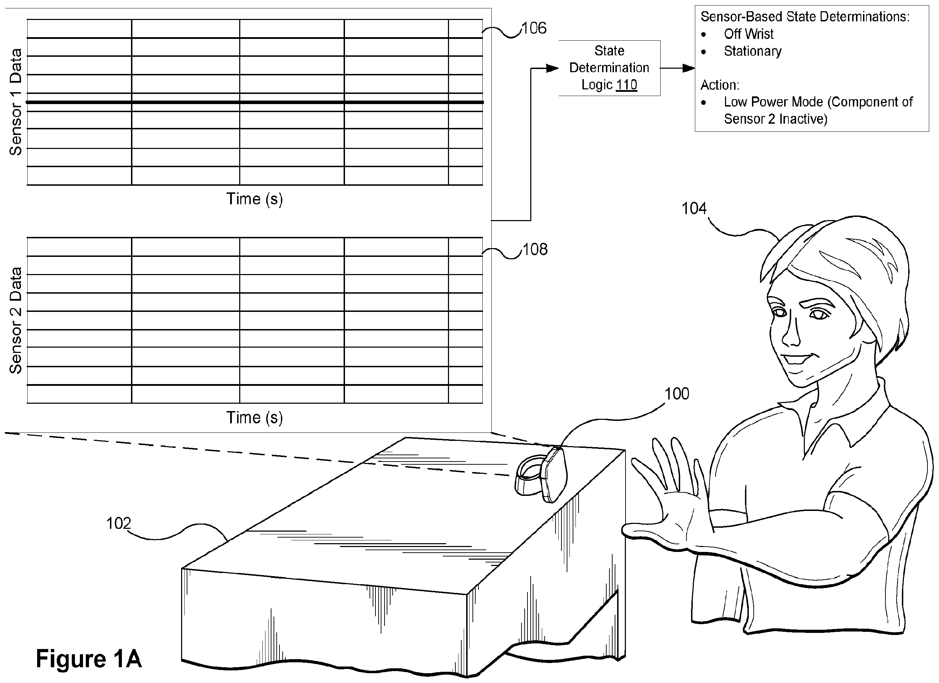

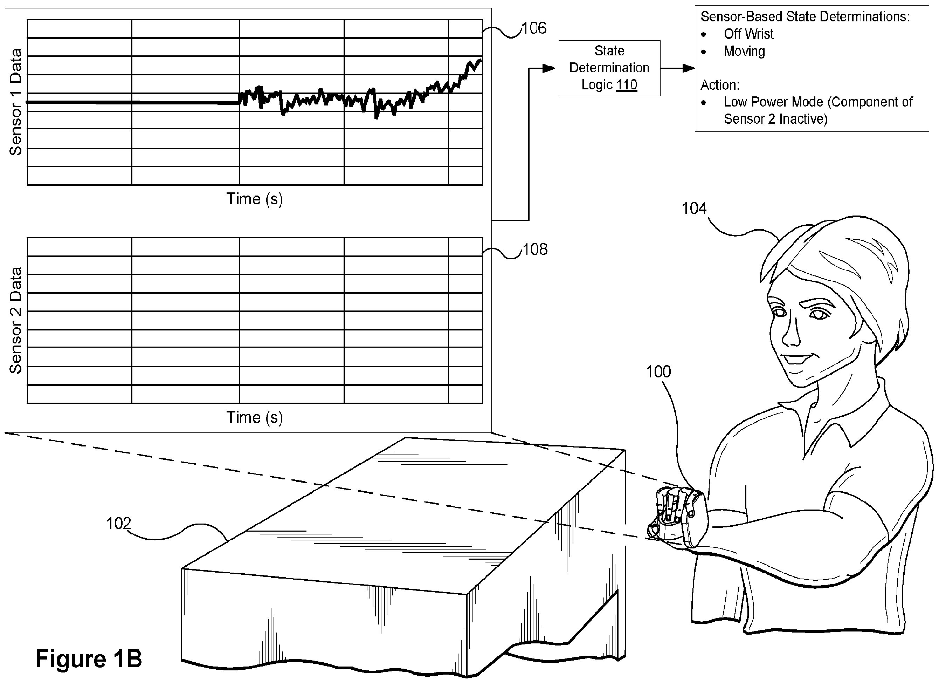

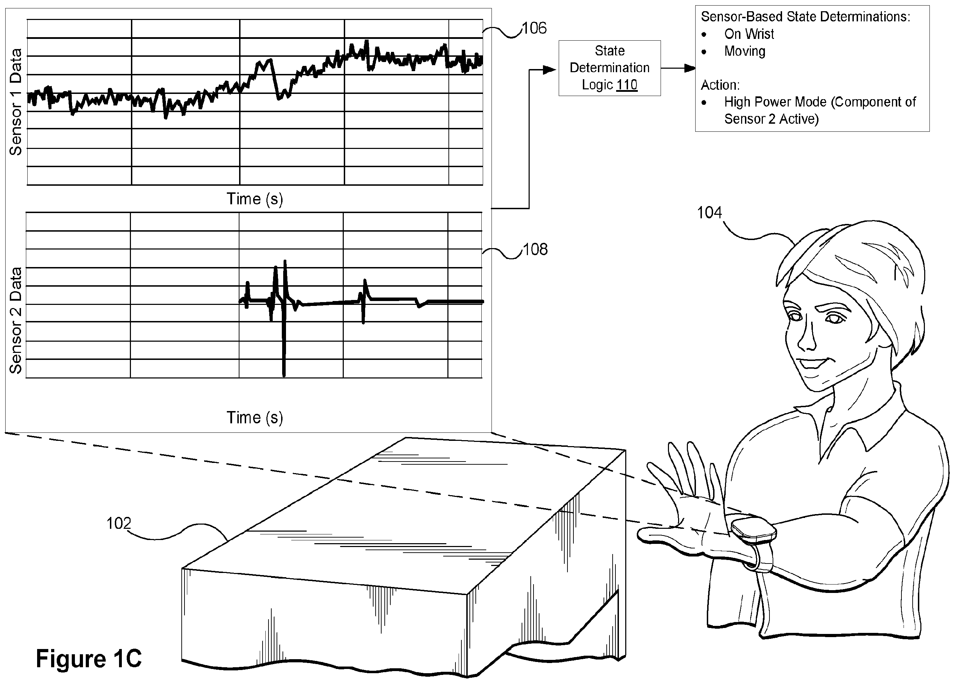

(A1) In accordance with some embodiments, a method of optimizing power consumption in a wearable device is performed at a wearable device (or at least a portion of the wearable device, such as a capsule portion that includes a display for the wearable device) that includes a first sensor configured to operate with a first power consumption rate and a second sensor configured to operate with a second power consumption rate that is greater than the first power consumption rate (e.g., the second sensor with the second power consumption rate can be referred to as a power-hungry sensor relative to the first sensor, because it requires more power than the first sensor to operate so is more power hungry than the first sensor). In one example discussed herein, the first sensor is part of an inertial measurement unit and the second sensor is part of a neuromuscular-signal sensor or group of neuromuscular-signal sensors. The method includes, while a component associated with the second sensor is in an inactive state (e.g., hardware and/or software associated with the second sensor can be inactive, including one or more of sensing electrodes, components used to process analog data from the sensing electrodes, and machine-learning models used to received processed data and determine gestures performed by a user): receiving, from the first sensor, first sensor data, and determining whether the first sensor data indicates movement of the wearable device (e.g., the example scenario of Figures 1A-1B shows that data from an IMU can be the first sensor data and it can indicate movement of the wearable device from a stationary, on-table position to a position in which the wearable device is held in the user's hand, and movement from the position in which the wearable device is held in the user's hand to a position in which the wearable device is worn on the user's wrist is shown from Figure 1B to Figure 1C). The method also includes, in accordance with a determination that the first sensor data indicates movement of the wearable device, operating the component of the second sensor in an active state in which the component associated with the second sensor is used to actively sense or process sensor data (e.g., Figure 1C shows that a component of Sensor 2 has been moved to an active state based on sensor-based state determinations for the depicted wearable device; while Figure 1C shows that the component of the Sensor 2 is moved to the active statement once a wrist-on state occurs, the component can, in some embodiments be moved to the active state once the wearable device is determined to no longer be stationary, and this is explained in more detail below). The method includes after activating the component associated with the second sensor, receiving, from the second sensor, second sensor data (e.g., the second sensor data can be neuromuscular-signal data). The method further includes, in accordance with a determination that the second sensor data indicates that the wearable device has been placed on a user's body, continuing to operate the component associated with the second sensor in the active state.

(A2) In some embodiments of A1, the first sensor is an inertial measurement unit (IMU) sensor (e.g., an accelerometer and/or gyroscope) that is configured to detect data indicating movements of the wearable device (e.g., including data used to measure or approximate spatial positioning, angular rates, and accelerations), and the second sensor is an electromyography (EMG) sensor that is configured to detect neuromuscular signals indicating muscular movements of a user.

(A3) In some embodiments of A1, the first sensor is a hall effect sensor (HES) that is configured to be used to check if a display portion of the wearable device is attached to or detached from a cradle portion of the wearable device, and the second sensor is a photoplethysmography (PPG) sensor that is configured to be used to detect changes in blood flow in a user.

(A4) In some embodiments of A1, the first sensor is a hall effect sensor (HES) that is configured to be used to check if a display portion of the wearable device is attached to or detached from a cradle, and the second sensor is an inertial measurement unit (IMU) sensor that is configured to detect data indicating movements of the wearable device (e.g., including data used to measure or approximate spatial positioning, angular rates, and accelerations).

(A5) In some embodiments of any one of A1-A4, the first sensor has a first power-consumption rate between 2 to 5 mW/s (milliwatts per second).

(A6) In some embodiments of any one of A1-A5, the second sensor has a second power-consumption rate between 7 to 17 mW/s (e.g., a higher power consumption rate than that of the first sensor, so the second sensor is a relatively power-hungry sensor at least as compared to the first sensor).

(A7) In some embodiments of any one of A1-A6, the component associated with the second sensor is operated in the active state for a period of time, and, after the period of time, the method further includes, while the component associated with the second sensor is in the inactive state: receiving, from the first sensor, new first sensor data, and determining whether the new first sensor data indicates movement of the wearable device. The method also includes, in accordance with a determination that the first sensor data indicates that the wearable device has not moved, continuing to operate the component of the second sensor in the inactive state. In other words, to preserve limited power and computing resources at the wearable device, when the second sensor is not needed (e.g., because the sensor-based state determinations described herein indicate that the wearable device is not being moved from a stationary to a wrist-worn state), then the component of the second sensor can remain inactive and avoid using up the limited power and computing resources when its sensing services are not required. In this way, data from the lower-power sensor (the first sensor) is used to determine when the higher-power sensor (the second sensor) should be activated.

(A8) In some embodiments of any one of A1-A7, the continuing to operate the component associated with the second sensor in the active state includes continuing to operate the component associated with the second sensor in the active state until a deactivation trigger is detected.

(A9) In some embodiments of A8, the deactivation trigger is detected when data from the first sensor indicates that the wearable device has been removed from the user's body.

(A10) In some embodiments of A8, the deactivation trigger is detected when data from the second sensor indicates that the wearable device has been removed from the user's body.

(A11) In some embodiments of any one of A1-A10, the determination that the second sensor data indicates that the wearable device has been placed on the user's body includes determining that the second sensor data, as compared to subsequent sensor data from the second sensor, reflects a change in data sensed by the second sensor.

(A12) In some embodiments of A11, the second sensor data indicates that one or more sensing channels of the second sensor are receiving data at or above a noise threshold, and the subsequent sensor data indicates that the one or more sensing channels of the second sensor are receiving data below the noise threshold.

(A13) In some embodiments of any one of A1-A12 operating the component of the second sensor in the active state includes causing an interrupt signal to be sent to the component associated with the second sensor to cause it to transition from the inactive to the active state.

(A14) In some embodiments of any one of A1-A13, determining whether the first sensor data indicates movement of the wearable device includes determining whether the movement is consistent with one or more known movements associated with the user placing the wearable device on the user's body.

(A15) In some embodiments of any one of A1-A14, the component associated with the second sensor is a system-on-a-chip configured to process the second sensor data.

(A16) In some embodiments of any one of A1-A15, the component associated with the second sensor is a machine-learning model (or a portion thereof) used to process and/or analyze the second sensor data.

(A17) In some embodiments of any one of A1-A16, the component is an electrode configured to sense the second sensor data.

(A18) In some embodiments of A17, the component includes a channel of at least two electrodes configured to sense the second sensor data.

(A19) In some embodiments of any one of A1-A18, the component includes both at least one electrode configured to sense the second sensor data and a machine-learning model configured to process the second sensor data.

(A20) In some embodiments of any one of A1-A19, the wearable device is a wrist-wearable device or a head-worn wearable device.

(A21) In some embodiments of any one of A1-A20, while the wearable device has been placed on the user's body: in accordance with a determination that a display portion (also referred to herein as a capsule portion) of the wearable device has been detached from a cradle, locking the wearable device until an authentication input is received. Similarly, in some embodiments, while the display portion of the wrist-wearable device is unlocked while it is worn on the user's wrist, the display portion of the wrist-wearable device can be configured to remain in the unlocked state until it is determined that the wrist-wearable device as a whole, or the display portion at a minimum, has been removed from the user's wrist.

(A22) In some embodiments of A21, the authentication input is a biometric input (including facial-recognition or fingerprint-recognition biometric inputs).

(A23) In some embodiments of A22, the biometric input is a predefined movement pattern of a finger of the user in front of a camera of the wearable device or an image of the finger of the user that is captured by the camera of the wearable device to detect at least one unique biometric characteristic of the finger.

(B1) In accordance with some embodiments, a wrist-wearable device is provided, and the wrist-wearable device is configured to perform or cause performance of the method of any one of A1-A23.

(C1) In accordance with some embodiments, a capsule configured to receive data from the first sensor and the second sensor recited in A1 is provided (in some embodiments, the first and second sensor can be housed in the capsule, while in other embodiments the first and second sensor can be coupled with the band or cradle portions of the wrist-wearable device and configured to send data to the capsule portion, and combinations are also contemplated in which some sensors can be housed in the capsule while others can be coupled to the band). In some embodiments, the capsule is configured to couple with a band to form a wrist-wearable device (e.g., through a detachable/ removable coupling via magnets or other suitable attachment mechanism to a cradle portion of the wrist-wearable device), and the capsule includes one or more processors configured to perform or cause performance of the method of any one of A1-A23.

(D1) In accordance with some embodiments, a non-transitory, computer-readable storage medium is provided. The computer-readable storage medium includes instructions that, when executed by a wrist-wearable device, cause the wrist-wearable device to perform or cause performance of the method of any one of A1-A23.

(E1) In accordance with some embodiments, a wrist-wearable device is provided that comprises means for performing or causing performance of the method of any one of A1-A23.

Having thus summarized the first aspect, the second aspect, which relates to techniques for optimizing when to use three different sensors of a wearable device to assist with determining cradle, wrist, and activity states of the wearable device, will now be summarized.

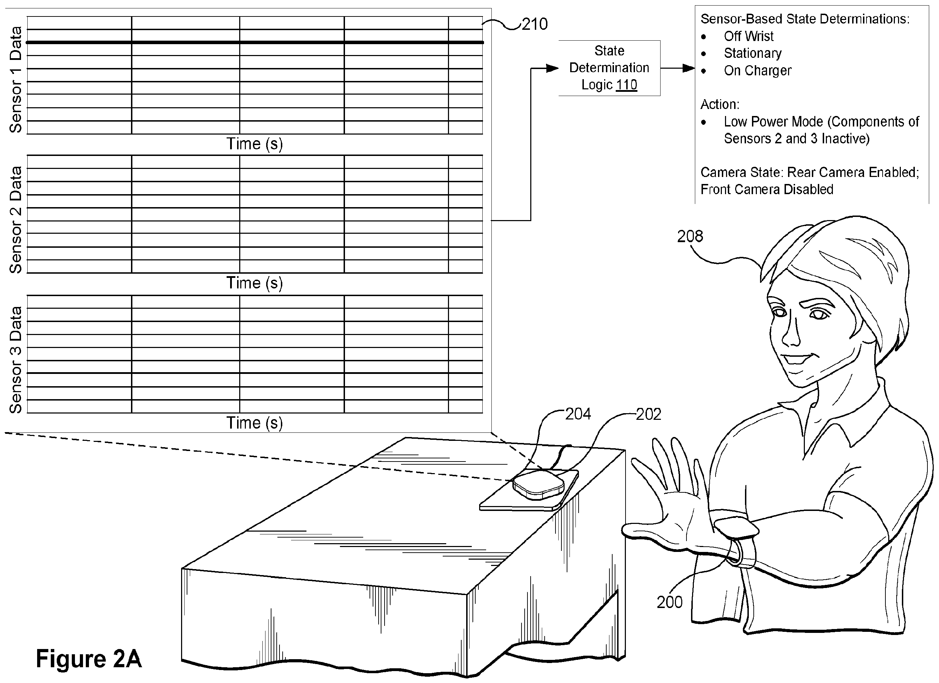

(G1) In accordance with some embodiments, a method of using three types of sensors to determine cradle, wrist, and activity states of a wrist-wearable device is provided. The method can be performed at a wrist-wearable device (the method can also be performed at other types of wearable devices that might include a detachable display portion, such as a head-worn device, ankle-worn device, finger-worn device, etc.) that includes a capsule portion that is configured to be removably coupled to a cradle portion of the wrist-wearable device, the wrist-wearable device including a first sensor of a first sensor type and a second sensor of a second sensor type distinct from the first sensor type. The method includes, determining, using sensor data from the first sensor of the first sensor type, whether the capsule portion of the wrist-wearable device is coupled with the cradle portion of the wrist-wearable device. The method includes, in accordance with a determination that the data from the first sensor of the first sensor type indicates that the capsule portion of the wrist-wearable device is coupled with the cradle portion of the wrist-wearable device, obtaining data from the second sensor of the second sensor type. The method also includes, in accordance with a determination that the data from the second sensor of the second sensor type indicates that the wrist-wearable device is worn on a wrist of a user, monitoring data from a third sensor of a third sensor type that is distinct from the first and second sensor types to determine an activity being performed by the user while the wrist-wearable device is worn on the user's wrist. In other words, a tiered sequence of determinations using the three types of sensors is utilized in order to conduct various sensor-based determinations concerning positional-based and use-based states of the wrist-wearable device; allocation of the three types of sensors to each of the determinations can be made to efficiently utilize the limited power and computing resources of the wrist-wearable device. The sequence shown in Figures 2A-2G depicts one example.

(G2) In some embodiments of G1, the first sensor is a hall effect sensor (HES) that provides data that is used to check if the capsule portion of the wrist-wearable device is coupled or decoupled from the cradle portion of the wrist-wearable device, and the second sensor is (i) photoplethysmography (PPG) sensor that provides data that is used to detect changes in blood flow in a user, (ii) electromyography (EMG) sensor that provides data that is used to determine muscular activities of a user, or (iii) an infrared (IR) sensor that provides data used to detect proximity to objects.

(G3) In some embodiments of any one of G1-G2, the method includes, after determining whether the capsule portion of the wrist-wearable device is on the wrist of the user, determining using a component of a third sensor whether the wrist-wearable device is being used during sleep or during exercise.

(G4) In some embodiments of any one of G1-G3, the third sensor is an inertial measurement unit (IMU) sensor that is configured to provide data used to approximate or measure inertial parameters associated with motion of the wrist-wearable devices, including, e.g., angular rates and acceleration of the wrist-wearable device, as well as (in some circumstances) orientation information for the wrist-wearable device as well as information from a magnetometer (the IMU can have multiple sensors, including an accelerometer, gyrometer, and magnetometer).

(G5) In some embodiments of any one of G1-G4, the method includes, in accordance with a determination that the third sensor indicates that the wrist-wearable device is being used during exercise, operating the second sensor at a first frequency; and in accordance with a determination that the third sensor indicates that the wrist-wearable device is being used during sleep, operating the second sensor at a second frequency that is higher than the first frequency.

(G6) In some embodiments of any one of G1-G5, the first frequency is between 15Hz and 50Hz, and the second frequency is between 100Hz and 150Hz.

(G7) In some embodiments of any one of G1-G6, the method includes, in accordance with a determination that the third sensor indicates that the wrist-wearable device is being used during sleep, activating a first subset of components of the second sensor, and in accordance with a determination that the third sensor indicates that the wrist-wearable device is being used during exercise, activating a second subset of components of the second sensor, which is a different subset of components than the first subset of components.

(G8) In some embodiments of any one of G1-G7, the first subset of components of the second sensor includes an IR sensor, a red light emitting diode (LED), and a green LED, and the second subset of components of the second sensor includes an IR sensor and a green LED.

(G9) In some embodiments of any one of G1-G8, the wrist-wearable device includes at least two cameras. The method further includes, in accordance with a determination that the third sensor indicates that the wrist-wearable device is being used during exercise, enabling a first camera of the at least two cameras, and in accordance with a determination that the third sensor indicates that the wrist-wearable device is being used during sleep, the method includes forgoing enabling the at least two cameras.

(G10) In some embodiments of any one of G1-G9, the at least two cameras include a front-facing camera and a rear-facing camera.

(G11) In some embodiments of any one of G1-G10, the method includes, in accordance with a determination that the data from the first sensor of the first sensor type indicates that the capsule portion of the wrist-wearable device is not coupled with the cradle portion of the wrist-wearable device, the wrist-wearable device enables the rear facing camera, and in accordance with a determination that the data from the first sensor of the first sensor type indicates that the capsule portion of the wrist-wearable device is coupled with the cradle portion of the wearable device, the wrist-wearable device enables the front facing camera.

(H1) In accordance with some embodiments, a wrist-wearable device is provided, and the wrist-wearable device is configured to perform or cause performance of the method of any one of G1-G11.

(11) In accordance with some embodiments, the capsule portion recited in G1 is provided as a standalone component, where the capsule portion is configured to couple with a cradle portion of a band to form a wrist-wearable device, and the capsule portion includes one or more processors configured to perform or cause performance of the method of any one of G1-G11.

(J1) In accordance with some embodiments, a non-transitory, computer-readable storage medium is provided. The computer-readable storage medium includes instructions that, when executed by a wrist-wearable device, cause the wrist-wearable device to perform or cause performance of the method of any of G1-G11.

(K1) In accordance with some embodiments, a wrist-wearable device is provided that comprises means for performing or causing performance of the method of any one of G1-G11.

Having thus summarized the second aspect, the third aspect, which relates to repurposing a metallic data-transmission component to be used as both a communication device and a device for detecting capacitance of a nearby surface or other object, will now be summarized

(L1) In accordance with some embodiments, a method of repurposing a metallic data-transmission component included in an electronic device (e.g., a wrist-wearable device, a head-worn device, or another type of wearable device, but other types of devices are also contemplated, including smartphones) is provided. The electronic device includes one or more processors. The method includes receiving, via the metallic data-transmission component, operational data indicative of a current operational state of the metallic data-transmission component. The method further includes determining, based on the operational data, whether data-transmission criteria (or a single data-transmission criterion) are (is) present. The method also includes, in accordance with a determination that the data-transmission criteria are present, operating the metallic data-transmission component in a first mode. In some embodiments, while in the first mode, the metallic data-transmission component is configured to be used as antenna in conjunction with transferring data between the electronic device and at least one other electronic device using the metallic data-transmission component (e.g., for purposes of conveying information using a near-field communication protocol, such as for activating a smart lock device or for touchless payment services). The method also includes, in accordance with a determination that the data-transmission criteria are not present, operating the metallic data-transmission component in a second mode, in which the metallic data-transmission component functions as a capacitive sensor (e.g., for touch-sensing purposes, which can also be used to assist with determining a positional state of a portion of a wrist-wearable device such as whether a capsule portion of the wrist-wearable device is attached to, or detached from, a cradle portion of the wrist-wearable device).

(L2) In some embodiments of L1, the electronic device includes one or more sensors. The method also includes receiving, via the one or more sensors, sensor data indicating a position of the metallic data-transmission component with respect to the electronic device, and determining whether data-transmission criteria are present based in part on the sensor data.

(L3) In some embodiments of any one of L1-L2, the one or more sensors include another capacitive sensor, and the method further comprises, while the metallic data-transmission component is operating in the second mode: the electronic device receives via the capacitive sensor a first capacitance value, and the electronic device receives via the other capacitive sensor a second capacitance value. The method also includes, while the metallic data-transmission component is operating in the second mode: the electronic device determines a true capacitance value based on a comparison of the first capacitance value and the second capacitance value, and the electronic device provides the true capacitance value to the electronic device to perform an action based on the true capacitance value.

(L4) In some embodiments of any one of L1-L3, the one or more sensors include another capacitive sensor that is disabled when the metallic data-transmission component is operating in the second mode. Some embodiments utilize only the metallic-data transmission component for capacitive sensing and thus do not have another capacitive sensor at all.

(L5) In some embodiments of any one of L1-L4, the metallic data-transmission component is coupled to a switch. In some embodiments, the electronic device provides a switching signal to the switch to selectively couple the metallic data-transmission component with (i) data-communication circuitry while the metallic data-transmission component is operating in the first mode and (ii) sensor-processing circuitry while the metallic data-transmission component is operating in the second mode based on a determination whether the data-transmission criteria are present.

(L6) In some embodiments of any one of L1-L5, capacitance detected via the metallic data-transmission component while it is operating in the second mode is used in conjunction with the methods of any one of A1-A23 or G1-G11.

(L7) In some embodiments of any one of L1-L6, the electronic device is a wrist-wearable device including a capsule and an accessory configured to couple with the capsule, and determining whether data-transmission criteria are present includes determining that the capsule is coupled to the accessory.

(L8) In some embodiments of any one of L1-L7, the electronic device includes a communications component for communicating with at least one other device, and determining whether data-transmission criteria are present is further based on one or more communication signals transferred between the electronic device and the at least one other device.

(L9) In some embodiments of any one of L1-L8, the data-transmission criteria includes one or more of a predefined idle time, predefined separation distance, predefined capacitance value, and predefined number of coupling attempts.

(L10) In some embodiments of L9, the predefined separation distance is equal to or less than 5 mm.

(L11) In some embodiments of any one of L9-L10, the predefined capacitance value is within a range of values between 100-200 pF.

(L12) In some embodiments of any one of L9-L1 1, the predefined idle time is a nonzero value that is equal to or less than 5 ms seconds.

(L13) In some embodiments of any one of L9-L12, predefined number of coupling attempts is a nonzero value that is equal to or less than three coupling attempts.

(L14) In some embodiments of any one of L1-L13, a wireless-communications component of the wrist-wearable device (e.g., a BLUETOOTH^{™} radio) operates at a first frequency and the metallic data-transmission component operates at a second frequency lower than the first frequency.

(L15) In some embodiments of any one of L1-L14, while operating the metallic data-transmission component in the second mode, receiving capacitance values at a first point and time and a second point in time, where a time between the first point in time and the second point in time is equal to or less than 300 ms. In other words, capacitance values sensed by the metallic data-transmission component are received at a sampling interval that is a nonzero value equal to or less than 300 ms.

(M1) In accordance with some embodiments, a wrist-wearable device is provided, and the wrist-wearable device is configured to perform or cause performance of the method of any one of L1-L15.

(N1) In accordance with some embodiments, a capsule portion, which includes the metallic data-transmission component recited in L1, is provided. In some embodiments, the metallic data-transmission component, is configured to couple with a band to form a wrist-wearable device, and the capsule includes one or more processors configured to perform or cause performance of the method of any one of L1-L15.

(O1) In accordance with some embodiments, a non-transitory, computer-readable storage medium is provided. The computer-readable storage medium includes instructions that, when executed by a wrist-wearable device, cause the wrist-wearable device to perform or cause performance of the method of any one of L1-L15.

(P1) In accordance with some embodiments, a wrist-wearable device is provided that comprises means for performing or causing performance of the method of any one of L1-L15.

Having summarized the above aspects, a brief description of the drawings will now be presented.

### BRIEF DESCRIPTION OF THE DRAWINGS

For a better understanding of the various described embodiments, reference should be made to the Detailed Description below, in conjunction with the following drawings in which like reference numerals refer to corresponding parts throughout the figures.
Figures 1A-1C illustrate a scenario in which a component of a sensor of a wearable device (e.g., Sensor 2 in the illustrated examples) is moved from an inactive to an active state based on sensor-based state determinations, in accordance with some embodiments.
Figures 1D-1E illustrate a scenario in which the component of the sensor is moved from the active state back to the inactive state based on sensor-based determinations indicated that the wearable device has been removed from the wrist of the user (which can occur upon the removal occur or at a later time such as when the wearable device is determined to have been placed back on a table), in accordance with some embodiments.
Figures 2A-2G illustrate a wearable device that includes three sensors (e.g., three sensors selected from: an inertial measurement unit (IMU) sensor, electromyography (EMG) sensor, hall effect (HES) sensor, photoplethysmography (PPG), infrared (IR) sensor, etc.) and these figures also illustrate scenarios under what conditions components of those sensors are placed in active or inactive modes, in accordance with some embodiments.
Figure 3 illustrates a state-transition diagram showing state transitions for a wearable device based on data from different sensors of the wearable device, in accordance with some embodiments.
Figure 4 illustrates a flow chart for how a wearable device, using state determination logic, places components of sensors in active or inactive states in response to the wearable device being removed from a wrist of the user, in accordance with some embodiments.

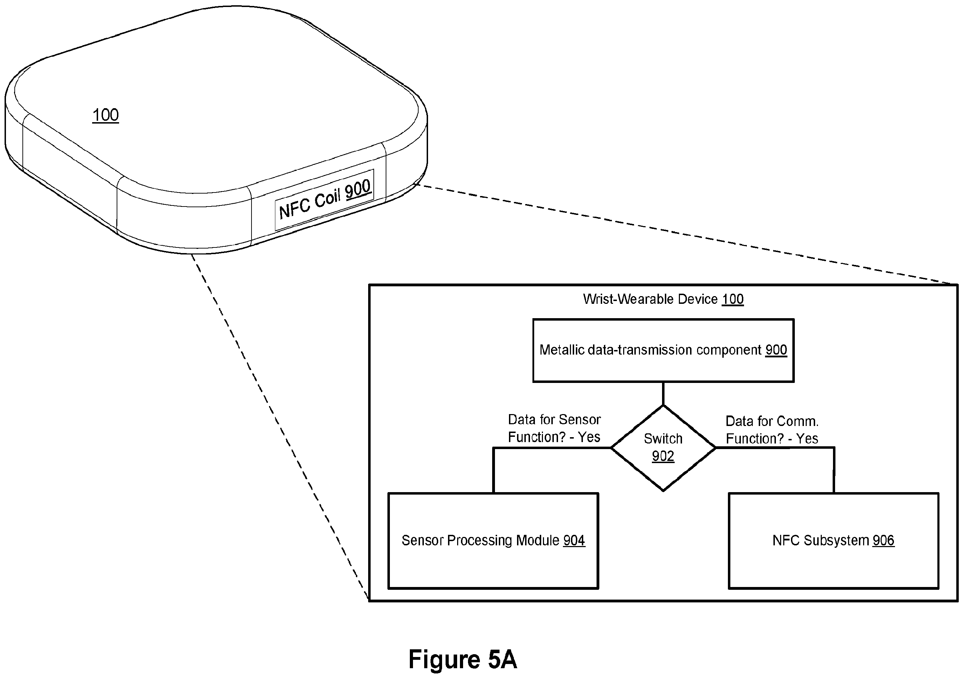

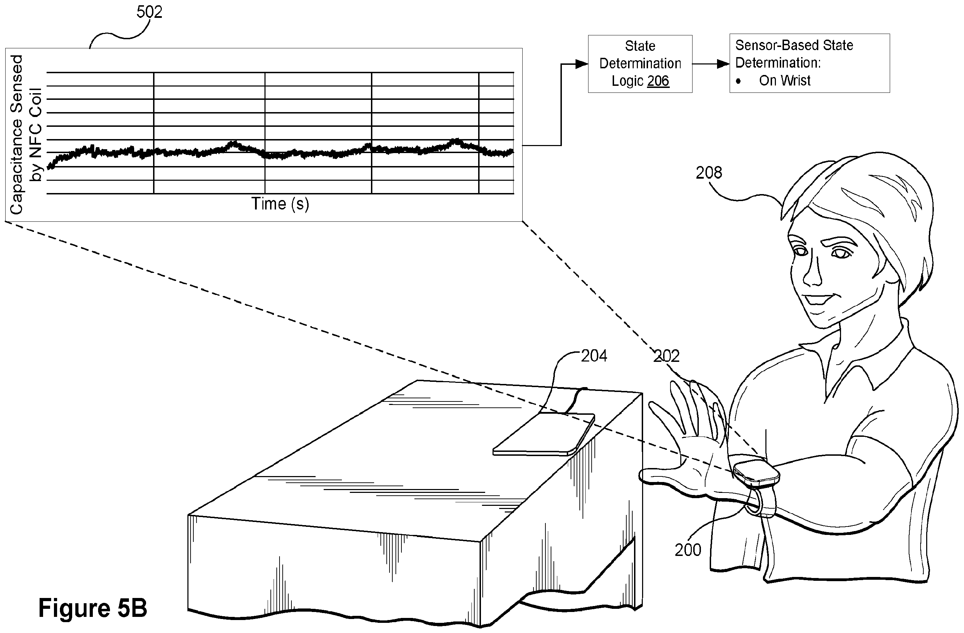

Figure 5A-5D related to repurposing of a metallic data-transmission component to serve multiple purposes, including a block diagram of a wrist-wearable device system and on-board logic for using a metallic data-transmission component, in accordance with some embodiments. In particular, Figure 5A illustrates a block diagram of a metallic data-transmission component (e.g., an NFC coil) that is configured to transmit data and also configured to detect capacitance of nearby surfaces or objects, in accordance with some embodiments. And Figures 5B-5D illustrate scenarios in which the metallic-data transmission component is used for the capacitance-sensing purpose, in accordance with some embodiments.
Figures 6A-6C illustrate an example wrist-wearable device, in accordance with some embodiments.
Figure 7 is a block diagram of a wrist-wearable device system, in accordance with some embodiments.
Figure 8 shows a block diagram of a representative computing system usable to implement the present disclosure, in accordance with some embodiments.
Figures 9A-9D illustrate an example flow chart of a method for activating and deactivating sensors of a wearable device, in accordance with some embodiments.
Figures 10A-10C illustrate an example flow chart of a method for placing components of sensors of a wearable device that includes three different sensors in active and inactive states, in accordance with some embodiments.
Figures 11A-11C illustrate an example flow chart of a method of repurposing a metallic data-transmission component (e.g., an NFC coil) in a wearable device to perform an additional function (e.g., a capacitive-sensing function), in accordance with some embodiments.
Figures 12A-12E illustrate a method of unlocking a wearable device using a gesture, in accordance with some embodiments.

In accordance with common practice, the various features illustrated in the drawings may not be drawn to scale. Accordingly, the dimensions of the various features may be arbitrarily expanded or reduced for clarity. In addition, some of the drawings may not depict all of the components of a given system, method or device. Finally, like reference numerals may be used to denote like features throughout the specification and figures.

### DETAILED DESCRIPTION OF EXAMPLES OF THE INVENTION

Reference will now be made in detail to embodiments, examples of which are illustrated in the accompanying drawings. In the following detailed description, numerous specific details are set forth to provide a thorough understanding of the various described embodiments. It will be apparent to one of ordinary skill in the art that the various described embodiments may be practiced without these specific details. In some instances, well-known methods, procedures, components, circuits, and networks have not been described in detail so as not to unnecessarily obscure aspects of the embodiments.

As will become apparent to a person of skill in this art upon reading this disclosure, the various embodiments provide systems and methods of providing optimized power (activating and deactivating sensors as needed to conserve power) consumption of a wearable device based on determined states of the wearable device.

An example method of optimizing power consumption in a wearable device, is performed at a wearable device (or at least a portion of the wearable device) that includes a first sensor configured to operate with a first power consumption rate and a second sensor configured to operate with a second power consumption rate that is greater than the first power consumption rate. The method includes, while a component associated with the second sensor is in an inactive state: receiving from the first sensor, first sensor data, and the wearable device determines whether the first sensor data indicates movement of the wearable device. The method also includes, in accordance with a determination that the first sensor data indicates movement of the wearable device, operating the component of the second sensor in an active state in which the component associated with the second sensor is used to actively sense or process sensor data. The method includes, after activating the component associated with the second sensor, receiving, from the second sensor, second sensor data. The method further includes, that in accordance with a determination that the second sensor data indicates that the wearable device has been placed on a user's body, continuing to operate the component associated with the second sensor in the active state.

Another example method uses three types of sensors to determine cradle, wrist, and activity states of a wrist-wearable device, and is performed at a wrist-wearable device that includes a capsule portion that is configured to be removably coupled to a cradle portion of the wrist-wearable device, the wrist-wearable device including a first sensor of a first sensor type and a second sensor of a second sensor type distinct from the first sensor type. The method includes, determining, using sensor data from the first sensor of the first sensor type, whether the capsule portion of the wrist-wearable device is coupled with the cradle portion of the wrist-wearable device. The method includes, in accordance with a determination that the data from the first sensor of the first sensor type indicates that the capsule portion of the wrist-wearable device is coupled with the cradle portion of the wrist-wearable device, obtaining data from the second sensor of the second sensor type. The method also includes, in accordance with a determination that the data from the second sensor of the second sensor type indicates that the wrist-wearable device is worn on a wrist of a user, monitoring data from a third sensor of a third sensor type that is distinct from the first and second sensor types to determine an activity being performed by the user while the wrist-wearable device is worn on the user's wrist.

In yet another example method, the method includes repurposing a metallic data-transmission component included in an electronic device, and the electronic device includes one or more processors. The method includes that the electronic device receives, via the metallic data-transmission component, operational data indicative of a current operation state of the metallic data-transmission component. The method includes, determining, based on the operational data, whether data-transmission criteria are present. The method includes, in accordance with a determination that the data-transmission criteria are present, operating the metallic data-transmission component in a first mode. In some embodiments, while in the first mode, the metallic data-transmission component is configured to be used as antenna in conjunction with transferring data between the electronic device and at least one other electronic device using the metallic data-transmission component. The method also includes, in accordance with a determination that the data-transmission criteria are not present, operating the metallic data-transmission component in a second mode, in which the metallic data-transmission component functions as a capacitive sensor.

Small portable devices, such as wearable devices, need to optimize their power usage to provide the best user experience, i.e., long periods of continuous use without needing a charge. The following figures illustrate how a wearable device activates and deactivates various sensors to improve battery life of small wearable devices.

Figures 1A-1E illustrate activating and deactivating sensors based on different determined activity states, in accordance with some embodiments. Figure 1A illustrates an interaction between a wearable device 100 resting on a table 102 (e.g., a non-biological surface) and a user 104. Figure 1A shows the wearable device 100 having at least two sensors (e.g., inertial measurement unit (IMU) sensor, electromyography (EMG) sensor, hall effect (HES) sensor, photoplethysmography (PPG), infrared (IR) sensor, etc.), as indicated by the chart 106 corresponding to data from a first sensor and chart 108 corresponding to data from the second sensor. As shown by chart 106, the first sensor is currently in a powered-on state, and is recording data indicative of the wearable device being in an inactive state. This inactive state is determined by state determination logic 110, which determines, using (only) the first sensor data, at least that the wearable device 100 is off the wrist of the user 104, the wearable device 100 is stationary. When these criteria are met, the wearable device 100 is kept in a low power mode. In some embodiments, the low power mode includes not powering on one or more sensors (e.g., the second sensor is powered off, which is indicated by no data being recording in chart 108). In some embodiments, the first sensor always remains powered on when the wearable device 100 is powered on.

Figure 1B illustrates that the user 104 has picked up the wearable device 100, but has yet to don the watch on their wrist. Using (only) the first sensor data as indicated by chart 106, the state determination logic 110 is configured to determine that the wearable device 100 is off the wrist of the user 104, and that the wearable device 100 is moving. Based on first sensor data, the wearable device 100 is still kept in a low power mode. In some embodiments, the low power mode includes not powering on one or more sensors (e.g., the second sensor is powered off, which is indicated by no data being recording in chart 108). In some embodiments, the device watches the first sensor data for activity for a predetermined amount of time before activating the second sensor, to confirm the device has been placed on the wrist of the user. Stated another way, the state determination logic 110 may determine the device is no longer stationary, but cannot confirm if the wearable device is on a wrist of the user until another sensor has been activated (e.g., a sensor for detecting whether the watch is in contact with a biological source).

Figure 1C shows that the user 104 has placed the wearable device 100 on their wrist. After a predetermined amount of time has elapsed and/or a movement threshold has been met, the second sensor, indicated by chart 108, is activated.

Using a combination of the first sensor data and the second sensor data, the state determination logic 110 is configured to determine whether the wearable device 100 is on the wrist of the user 104. In accordance with a determination by the state determination logic 110 that the wearable is on the wrist of the user 104, the wearable device 100 continues to keep the second sensor activated. In some embodiments, having the second sensor activated is referred to as a high-power mode. In some embodiments, the high-power mode includes activating one or more sensors that draw more power than other sensors. In some embodiments, the low power sensor (e.g., the first sensor) can be configured to shutoff when the higher-power sensor (e.g., the second sensor) is in use and would otherwise be supplying redundant data.

While Figures 1A-1C illustrate a process of confirming the first sensor data that indicates the wearable device 100 is on a wrist of a user, using second sensor data, however, it is also possible to activate the second sensor at different times. For example, in another embodiments, any kind of data received from the first sensor (e.g., for a period of time) can be sufficient for activating the second sensor, which is used to determine if the wearable device 100 is on a wrist of a user.

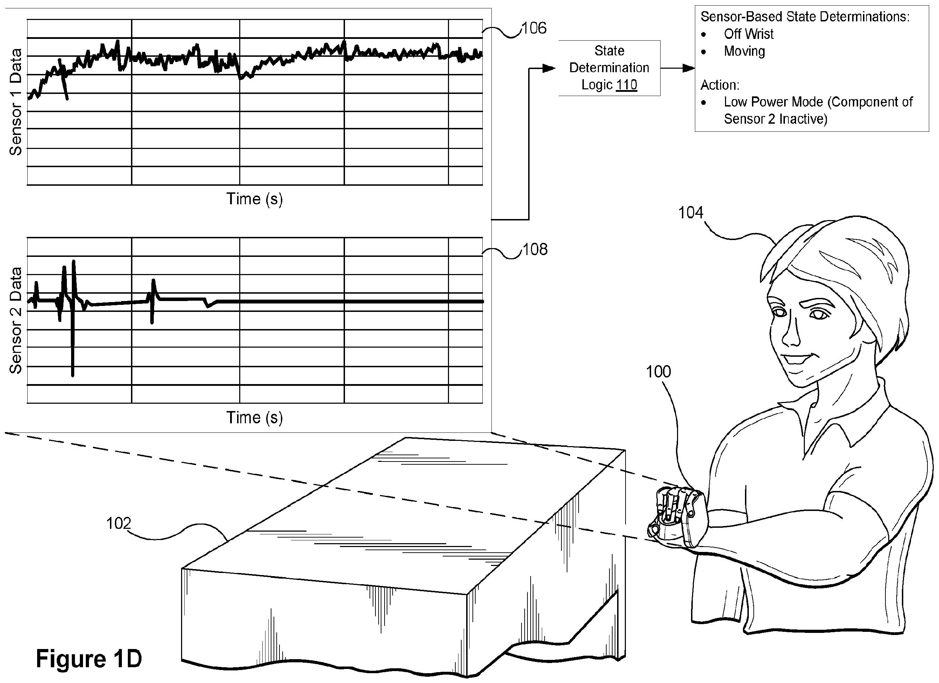

Figures 1D-1E illustrate a process for removing the wearable device 100 from the wrist of the user 104 and placing it back on the table 102, in accordance with some embodiments. Figure 1D shows that the user 104 has removed the wearable device 100 from their wrist, but is holding it in their hand. As a result, the second sensor is no longer able to retrieve the same data it was previously receiving (e.g., biometric information), as indicated by chart 108 showing a flat line. In some embodiments, the device watches the second sensor data for inactivity for a predetermined amount of time before deactivating the second sensor. In other words, when there is a lack of data coming from the second sensor (e.g., indicating the wearable device is off the wrist of the user 104), it remains powered on for a predetermined amount of time before determining it is no longer being used. For example, user 104 may be adjusting the wearable device 100 on their wrist, but not completely removing it. Thus, using a combination of the first sensor data and the second sensor data, the state determination logic 110 is configured to determine that the wearable device 100 is off the wrist of the user 104, the wearable device 100 is moving.

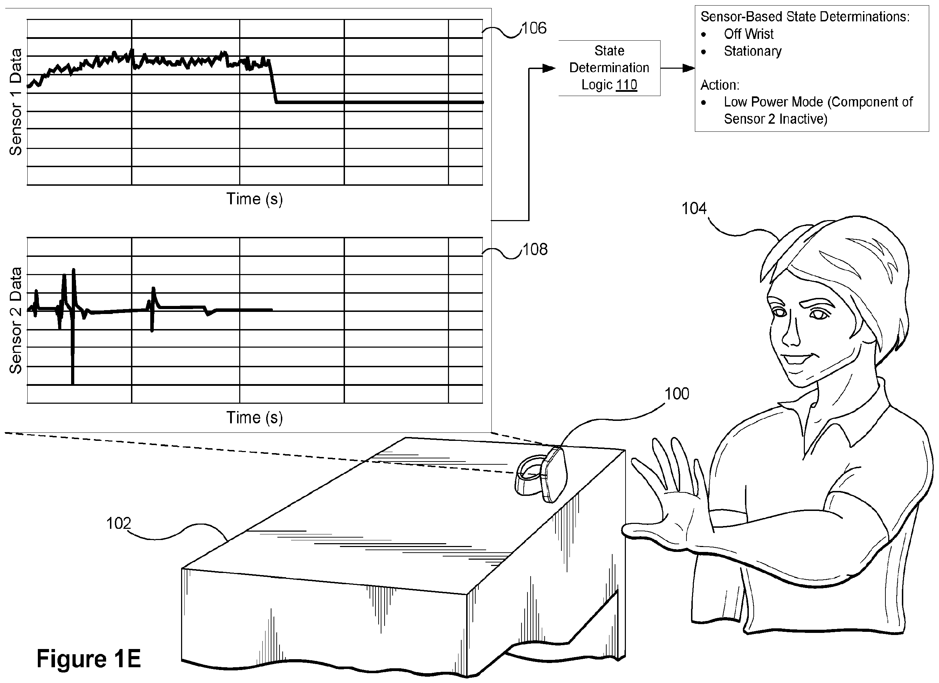

Figure 1E shows that the user 104 has placed the wearable device 100 on the table 102 and is no longer moving. Chart 108 also shows that the second sensor has been placed in a low power mode, as the wearable device 100 has determined it is no longer needs to be activated while being placed on a table 102 (e.g., off the wrist of a user). Using a combination of the first sensor data and/or the second sensor data, the state determination logic 110 is configured to determine that the wearable device 100 is off the wrist of the user 104 aid is stationary. In accordance with a determination that the wearable device is off the wrist of the user and is stationary, the wearable device 100 switches to a low power mode, that includes powering off the second sensor to conserve battery of the wearable device 100. In some embodiments, the low power mode operates at a consumption rate between 2-5 mW/s and the high-power mode operates at 5-17 mW/s.

While Figures 1D-1E powering off the second sensor when it is put on the table, it is also possible that the second sensor is deactivated at a different time. For example, the second sensor can be deactivated when the wearable device 100 is removed from the wrist of the user. In other words, the second sensor can be deactivated while being in the hand of the user, but not yet placed on non-biological surface (e.g., a table).

Figures 2A-2D illustrate a wearable device 100 that includes three sensors (e.g., three sensors selected from: an inertial measurement unit (IMU) sensor, electromyography (EMG) sensor, hall effect (HES) sensor, photoplethysmography (PPG), infrared (IR) sensor, etc.,) and under what conditions they are activated or deactivated, in accordance with some embodiments. Figure 2A illustrates that the wearable device 100 is composed of two parts a cradle 200 and a watch capsule 202, collectively referred to as the wearable device 100. As shown in Figure 2A, the watch capsule 202 is disconnected from the cradle 200 that is presently on the wrist of the user. While the watch capsule 202 is not connected to the cradle 200, it can be placed on a charging pad 204. As shown in Figure 2A, while the watch capsule 202 is on the charging pad 204, the watch capsule 202 using its state determination logic 206 to determine that the watch capsule 202 is off the wrist of a user 208, the watch capsule 202 is stationary, the watch capsule 202 is on the charging pad 204. In accordance with a determination that the watch capsule is capsule is off the wrist of a user 208, (i) the watch capsule 202 is stationary, (ii) and/or the watch capsule 202 is on the charging pad 204, the watch capsule operates in a low power mode (e.g., one or more sensors of the watch capsule and/or cradle are not in a powered-on state). Figure 2A also shows that, in chart 210, that a constant data point (or a specific pattern) is being recorded by the watch capsule 202, which indicates that the watch capsule 202 is placed on the charging pad 204 (e.g., a hall effect sensor indicates that a magnetic field is present and constant near the watch capsule 202).

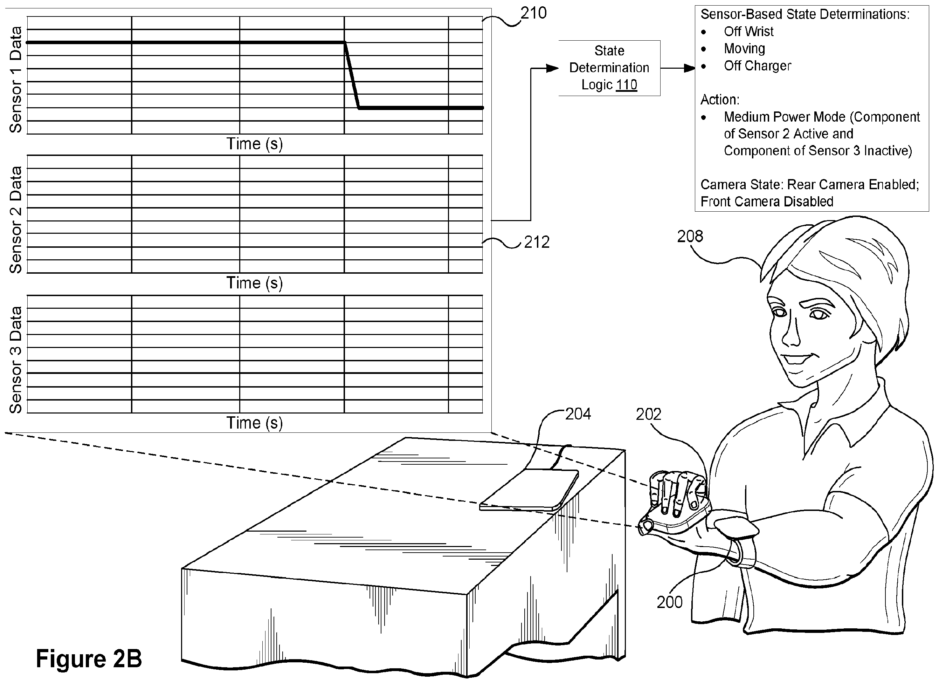

Figure 2B illustrates the user 208 picking up the watch capsule 202, but not yet attaching the watch to cradle 200. Chart 210 indicates that in response to the capsule being removed from the charging the pad, the data being recorded from the first sensor has changed. Using the state determination logic, the watch capsule determines, based at least on the data from the first sensor, that the watch capsule 202 is off the charging pad 204. In response to determining that the watch capsule 202 is off the charging pad 204, the watch capsule activates the second sensor, indicated by chart 212, which can be configured to determine whether the watch capsule 202 is placed on a wrist of a user 208. As shown in chart 212, the second sensor is powered on (e.g., a medium power mode) and is outputting data (e.g., one of movement data, proximity data, or biometric data). Using data from at least the first and/or second sensor the state determination logic 206 is able to determine that (i) the watch capsule is off the wrist of a user 208, (ii) the watch capsule 202 is moving, and (iii) the watch capsule 100 is off the charging pad 204.

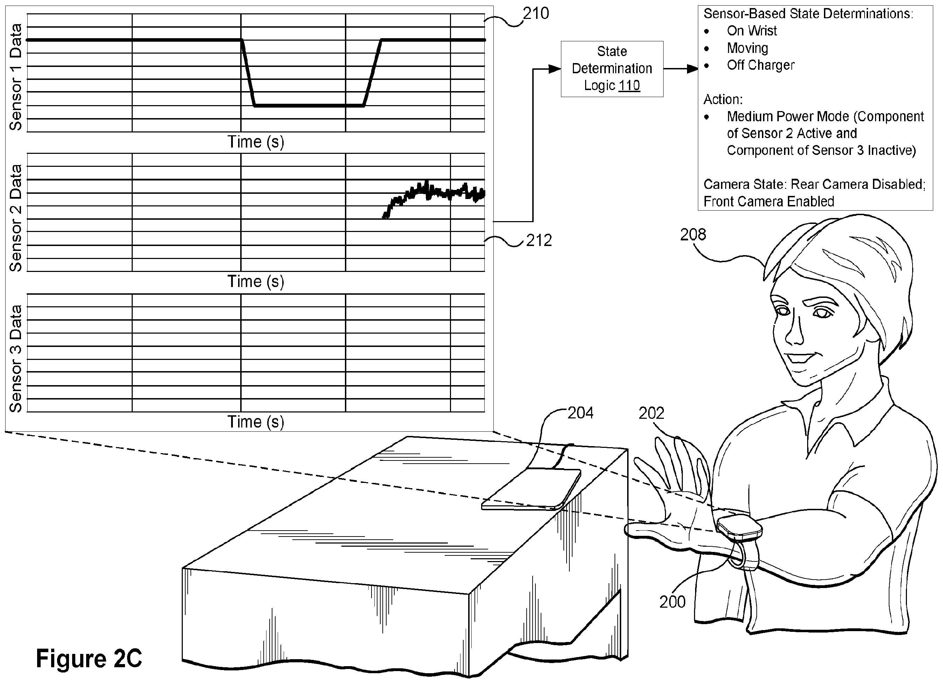

Figure 2C illustrates the user having connected the watch capsule 202 to the cradle 200. The first sensor, as illustrated by chart 210, is configured to provide data that the state determination logic 206 can use to determine if the watch capsule 202 has been placed on the cradle. Figure 2C shows that in accordance with a determination that first sensor provides data that indicates that the watch capsule 202 is connected to the cradle 200, the state determination logic 206 provides instructions for activating the second sensor to confirm whether the capsule 202 and cradle 200 are on a wrist of a user 208.

In some embodiments, the chart 210 shows another magnetic field being detected by a hall effect sensor. In some embodiments, the hall effect sensor is relied on to improve the security of the wearable device, by locking the device once the magnetic field is no longer present (e.g., when the capsule portion of the wearable device is detached from the cradle portion of the wearable device). By immediately locking, it ensures that if someone were to steal the capsule portion, they would not be able to access data, unless they unlocked the capsule device by providing authenticating information.

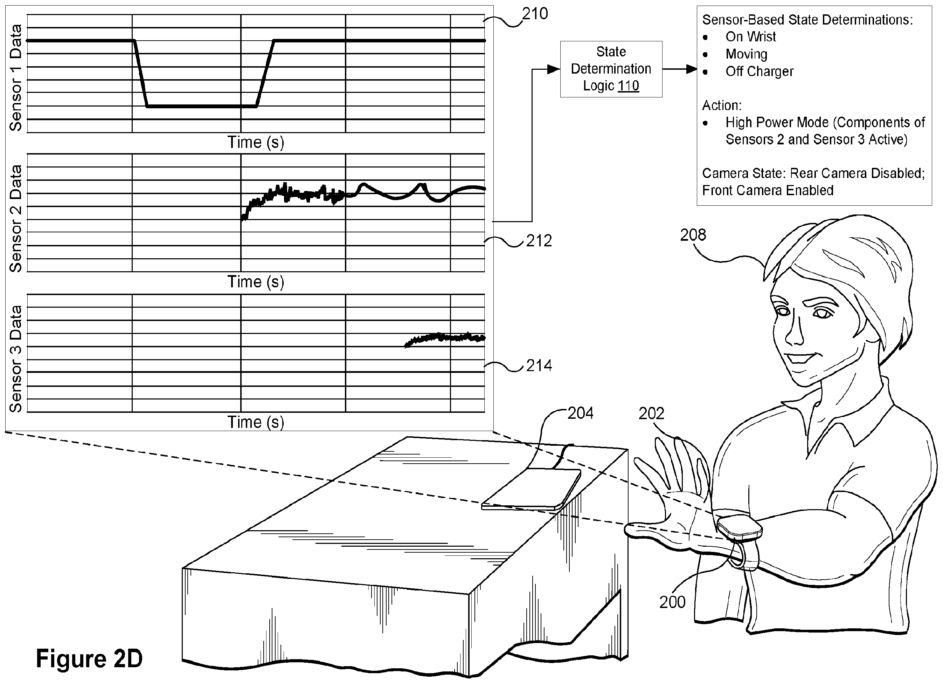

Figure 2D shows the user 208 having connected the watch capsule 202 to the cradle 200, and the chart 212 indicating a pattern associated with capsule 202 to the cradle 200 being on the wrist of the user. In other words, chart 212 shows that the second sensor records a different pattern when the watch capsule 202 is on the wrist of the user 208 and/or connected to the cradle 200 than when the watch capsule 202 is not on the wrist of the user 208 and/or not connected to the cradle 200. Using data from at least the first and/or second sensor, the state determination logic 206 determines: (i) that the watch capsule 202 is on the wrist of a user 208, (ii) the watch capsule 202 is moving, and (iii) the watch capsule 202 is off the charging pad 204. In response to the state determination logic 206 determining that the watch is on the wrist of the user, the state determination logic 206 provides instructions for activating a third sensor, as indicated by the data in chart 214 being recorded. In some embodiments, a third sensor is configured to detect an activity being performed by the user (e.g., exercising, sleeping, etc.). In some embodiments, the third sensor is an inertial movement unit (IMU).

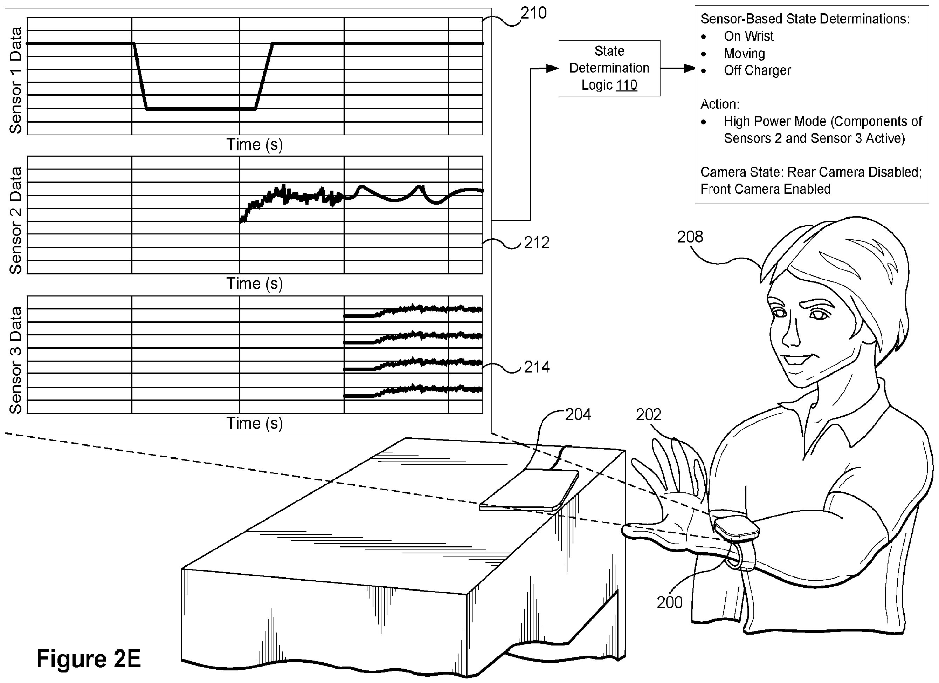

Figure 2E shows an alternative configuration, where the third sensor is an electromyography (EMG) sensor that measure electrical signals generated by a user. As indicated by the data in chart 214, there can be multiple sensors that have their data compiled together (e.g., multiple electrodes).

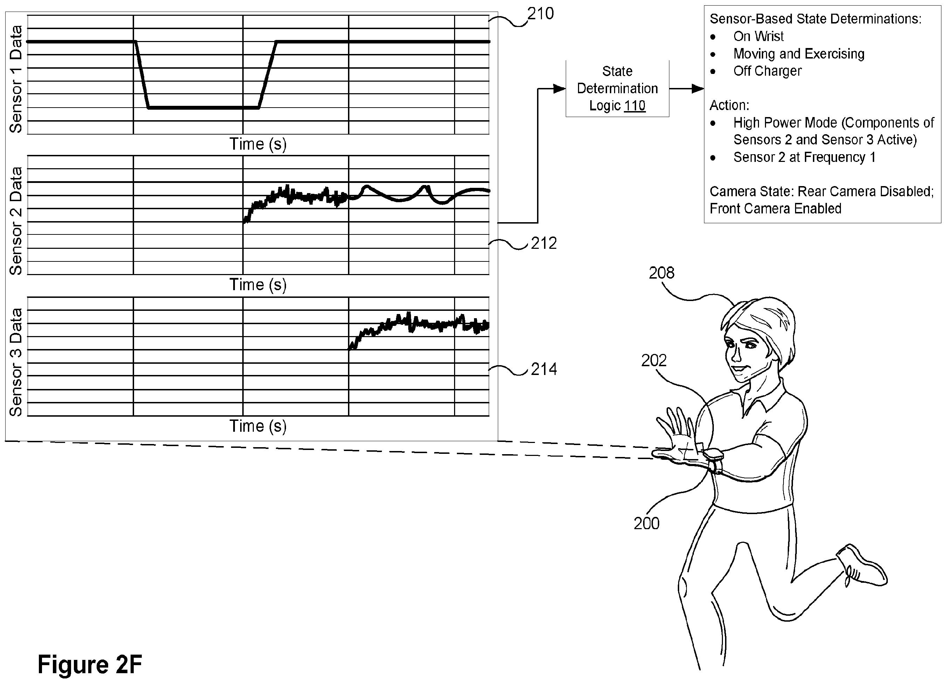

Figures 2F-2G illustrate that in some embodiments, the third sensor provides data for determining whether the user is performing a certain activity (e.g., exercising or sleeping).

Figure 2F illustrates that the state determination logic 206 has determined that the user has begun exercising, based at least on the data from the three sensors. Figure 2F also shows that in accordance with a determination that the user is exercising the second sensor operates at a first frequency (e.g., 15Hz and 50Hz).

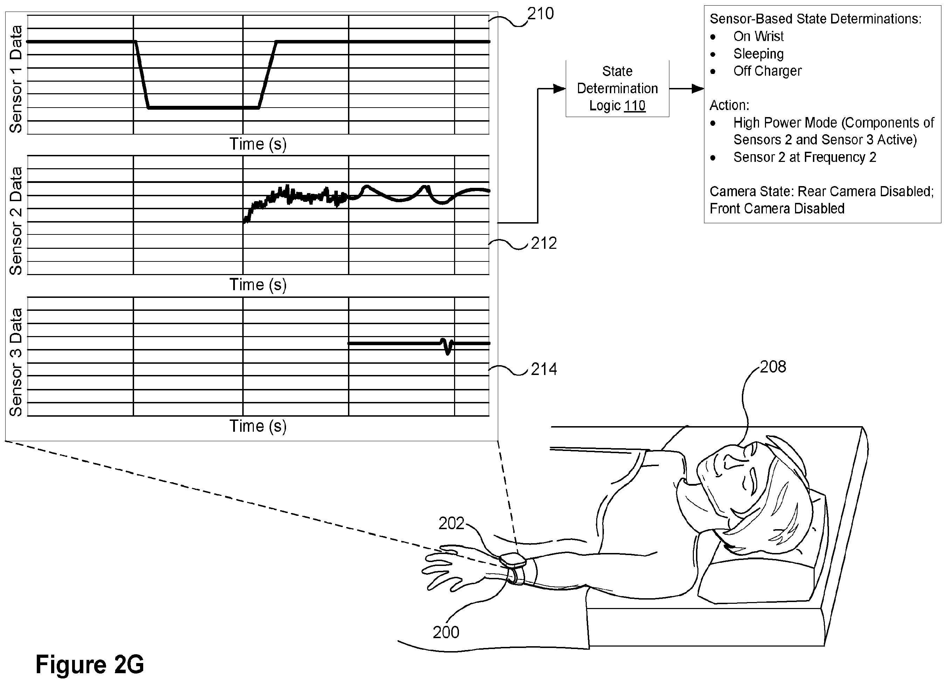

Figure 2G shows that the state determination logic 206 has determined that the user is sleeping, based on at least data recorded by the one or more sensors. In some embodiments, the second and third sensor provide data for determining whether the user is performing a certain activity (e.g., exercising or sleeping). Figure 2G also shows that in accordance with a determination that the user is sleeping the second sensor operates at a second frequency (e.g., 100Hz and 150Hz).

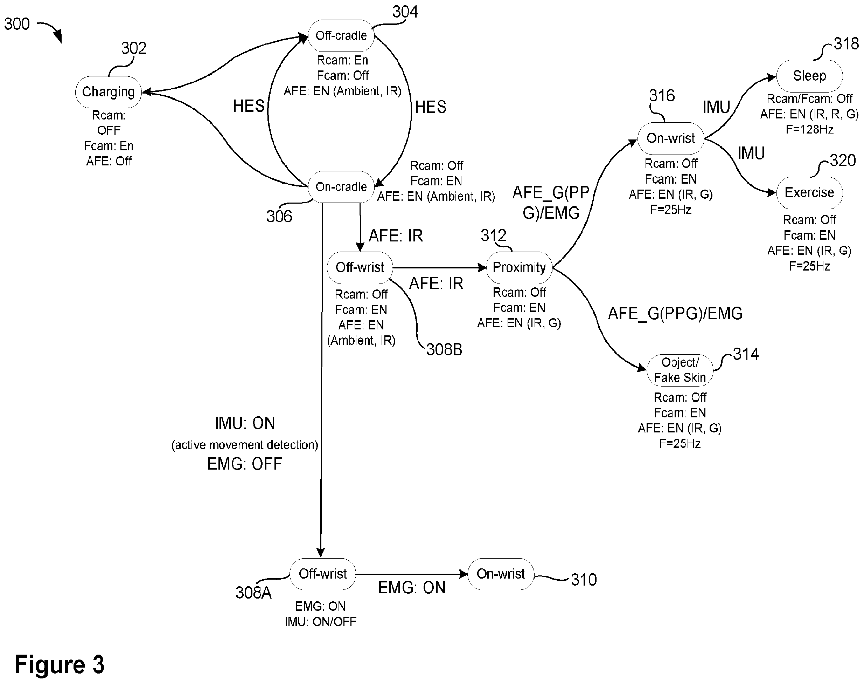

Figure 3 illustrates a flow chart 300 for when to enabled sensors on a wearable device (e.g., wearable device 100 or cradle 200 and a watch capsule 202) based on the devices activity status, in accordance with some embodiments. Figure 3 shows, in step 302, that when the wearable device is on a charging pad, the wearable device using state determination logic has the rear-facing camera powered off, the front-facing camera powered on, and an optional analog front-end (AFE) powered off.

Figure 3 also shows that the wearable device uses data from an optional hall effect sensor (HES) to determine if the device is on or off the cradle (e.g., a cradle 200). When the hall-effect sensor provides data indicating that the HES is off the cradle, as shown in step 304, the logic controlling the wearable device activates the rear-facing camera, deactivates the front-facing camera, and enables an optional AFE sensor, specifically the ambient light sensor and infrared sensor of the AFE.

When the hall-effect sensor provides data indicating that the HES is on the cradle, as shown in step 306, the logic controlling the wearable device deactivates the rear-facing camera, enables the front-facing camera, and AFE remains (the ambient light sensor and infrared sensor of the AFE remain enabled).

Alternatively, when a wearable device is not equipped with a HES, it can rely on inertial measurements sourced from an inertial measurement unit to determine if the wearable device is on or off the cradle.

Step 308A and 308B illustrate two possible paths the logic of the wearable device can follow, based on the configuration of the wearable device. Step 308A illustrates a first path that the state determination logic follows when a device is equipped with an IMU sensor and an EMG sensor, but not a AFE or a HES. Step 308B illustrates another branch of logic for a wearable device that includes an AFE and a HES.

In step 308A, the state determination logic uses IMU sensor information to detect active movement of the wearable device to determine whether the watch is on or off the wrist of a user. In some embodiments, the IMU data corresponds to a predetermined pattern that indicates a wrist off state and/or an off-charger state. After the state determination logic has determined that the wearable device is off wrist of a user, using data from the IMU sensor, the state determination logic activates the EMG. Using data from the activated EMG, the state determination logic determines, as shown in step 310, that the wearable device is on a wrist of a user.

Step 308B illustrates another branch of logic for a wearable device that includes an AFE and a HES. As shown in step 308B, when the data from the HES indicates that is off cradle, the state determination logic initiates an infrared (IR) sensor to determine if the watch is proximate to a wrist of a user.

Step 312 shows that when the state determination logic determines, using data from the IR sensor, that the wearable device is proximate to something, the state determination logic causes a PPG and/or EMG sensor to be activated. The PPG and/or EMG sensors are capable of providing data that can be used to determine if the wearable device is on a wrist of a user.

After step 312, the state determination logic can follow two branches depending on what the data from the PPG and/or EMG sensor indicate. Step 314 indicates the first logical branch, which is followed when the state determination logic determines, using data from the PPG and/or EMG sensor to determine if the wearable device is proximate to a wrist or something else (e.g., a tabletop, floor, desk mat (e.g., a surface that could be interpreted as wrist, unless a biometric sensor is used to confirm), etc.). Once the state determination logic determines, using data from the PPG and/or EMG sensor, that the wearable device is on a surface other than a wrist of the user, it forgoes any further operation until it detects that is on a wrist of a user.

Step 316 indicates the second logical branch, which is followed when the state determination logic determines, using data from the PPG and/or EMG sensor(s), that the wearable device is on a user's wrist. When the wearable device is on the wrist of the user, the PPG and/or EMG sensor(s) remain operational, and track biometric data. In some embodiments, the sensors run at frequency of 25Hz. In some embodiments, the frequency is variable depending on determined activity states and runs between 20Hz-200Hz.

After it has been determined that the wearable device has been place on a wrist of a user, the state determination logic then determines, using data from an IMU sensor, an activity being performed by a user. As shown in step 318, when the IMU sensor provides data that indicates the user is sleeping (e.g., the data provided by the IMU corresponds to a predetermined pattern that indicates a user wearing the wearable device is sleeping), the rear-facing camera is disabled, the front-facing camera is disabled, the red LED, the green LED, and the IR on the AFE are enabled, and the sensors are configured to operate at 128Hz. Step 320 indicates another logical branch using the IMU sensor. When the IMU sensor provides data that indicates that the user is exercising (e.g., the data provided by the IMU corresponds to a predetermined pattern that indicates a user wearing the wearable device is exercising), one or more sensors are operated at 25Hz, the green LED, and the IR on the AFE are enabled.

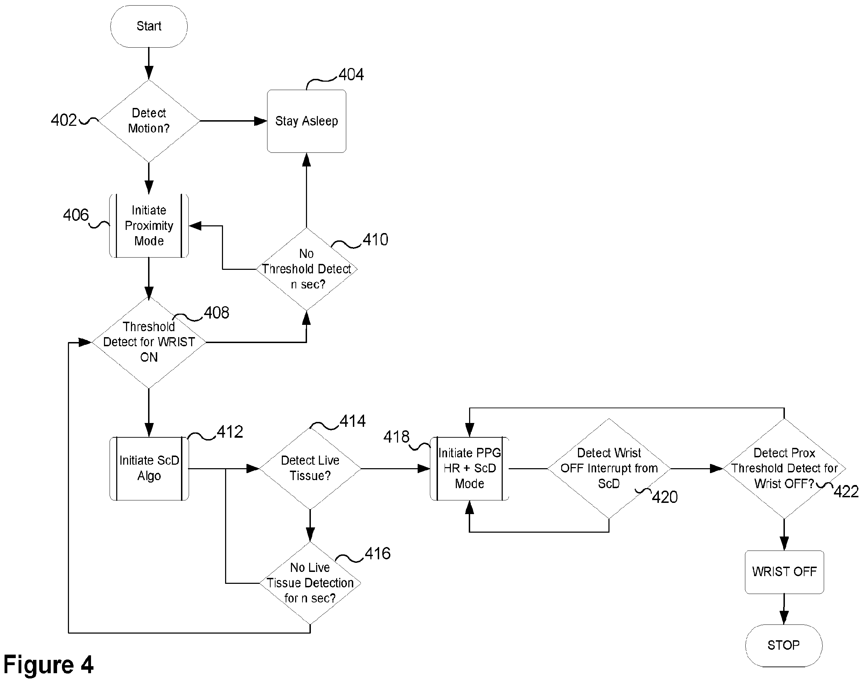

Figure 4 illustrates a flow chart 400 for how a wearable device, using state determination logic, activates and deactivates sensors in response to the wearable device being removed from a wrist of the user, in accordance with some embodiments. Figure 4 starts with first detecting motion 402, using one or more sensors (e.g., an IMU sensor). Figure 4 shows at step 404, in accordance with a determination that the no motion is detected (or a motion level that does not exceed a threshold amount of movement) the wearable device remains asleep. Step 406 shows that, in accordance with a determination that motion is detected (e.g., motion meets or exceeds a minimum motion threshold) the wearable device activates its proximity mode for determining whether the wearable device is proximate to a possible wrist and/or another surface. Step 408 then checks whether a wearable device has been proximate to a possible wrist and/or other surface for threshold amount of time. Step 410 indicates the procedure for the state determination logic to follow when the wearable device has not been proximate to a possible wrist and/or other surface for threshold amount of time. The wearable device is configured to either (i) revert to a sleep mode, or (ii) reinitiate the proximity mode. In some embodiments, the device is put to sleep when no surface is detected in the proximity mode, and the device reinitiates the proximity mode when the wearable device detects a surface for some amount of time, but does not meet or exceed the threshold amount of time.

Figure 4 shows at step 412 that, in response to the wearable device being proximate to a possible wrist and/or other surface for threshold amount of time, the state determination logic initiates the ScD algorithm to detect live tissue (e.g., a wrist of a user). In some embodiments, the ScD algorithm is illustrated by the flow chart shown in Figure 3. Step 414 illustrates possible steps to follow when live tissue is detected. First, if no live tissue is detected, as indicated in step 416, then the state determination logic returns to step 408 (described above). In some embodiments, if live tissue is detected, but does not meet or exceed the minimum time threshold, the wearable device is configured to check for live tissue again (i.e., repeat step 414). Step 418 shows that, in accordance with a determination that the live tissue is detected (e.g., live tissue is detected for a time period long enough to meet or exceed a time threshold), the state determination logic is configured to initiate a PPG heart rate monitor sensor, and s ScD mode.

The following steps in Figure 4 describe how the wearable device detects and handles the wearable device being removed from the wrist of a user. Step 420 shows that the wearable device relies on the ScD to detect whether the wearable device is off the wrist of the user. Step 420 also shows that, in accordance with a determination that the wearable device is not off the wrist of the user, the wearable device continues to operate the PPG heart rate sensor (i.e., reverts back to step 418).

Step 422 indicates that a proximity sensor is used to confirm whether an interrupt in receipt of biometric information from a biometric sensor is a wrist off event. In accordance with a determination that the interrupt occurs, and the proximity sensor indicates that the wearable device is not proximate to a surface (e.g., is beyond a threshold distance away from a surface), the state determination logic confirms that the wearable device is off the wrist of a user. Step 422 also shows that in accordance with a determination that the interrupt occurs, and the proximity sensor indicates that the wearable device is proximate to a surface (e.g., is not beyond a threshold distance away from a surface), the state determination logic confirms that the wearable device is still potentially on the wrist of a user and reverts to step 418.

Figure 5A-5C illustrate using a metallic data-transmission component (e.g., an NFC coil) that is configured to transmit data and also configured to detect capacitance of nearby surfaces, in accordance with some embodiments.

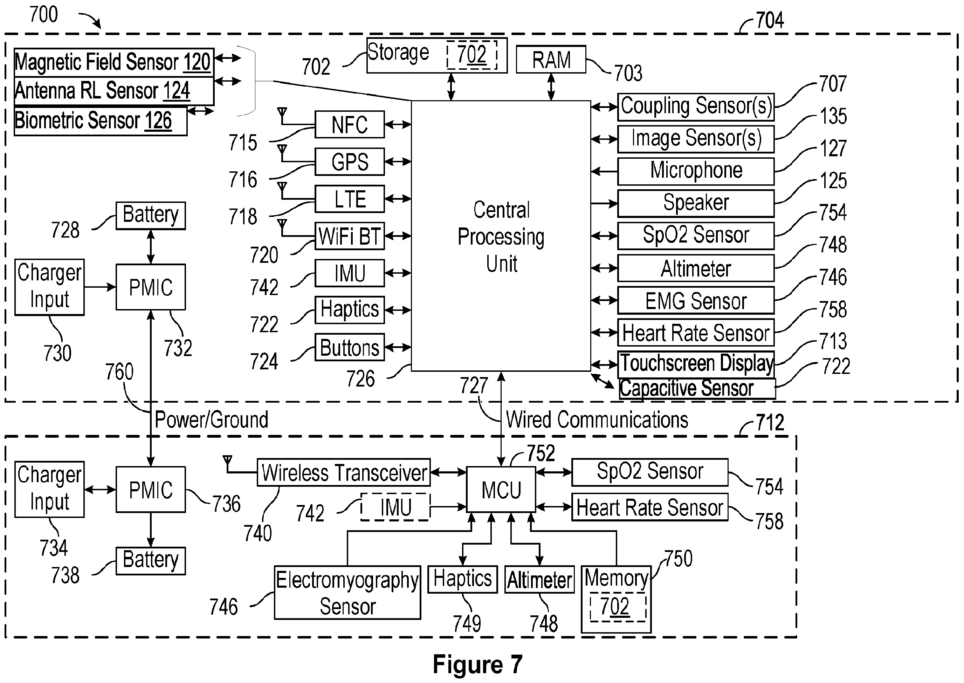

Figure 5A is a block diagram of a wrist-wearable device system 100 and on-board logic for using a metallic data-transmission component, according to at least one embodiment of the present disclosure. The device 100 includes a metallic data-transmission component 900 (e.g., a communication device, such as an NFC 715, discussed in reference to Figure 7). The metallic data-transmission component 900 can be configured to provide a dual purpose. The first purposed being to communicate as a near field communication component, and the second purposes being a component for detecting capacitance of a surrounding object (e.g., Figures 5A-5C illustrate an example of how the wearable device detects capacitance using the metallic data-transmission component 900 to determine whether the wearable device is on a wrist, off a wrist, or on a hand of a user).

Figure 5A also includes a switch 902 for connecting sensor processing module 904 to the metallic data-transmission component 900 to be used as a capacitive sensor. Alternatively, the switch 902 also connects to NFC subsystem 906 to use the metallic data-transmission component 900 as a near-field communication radio.

Figure 5B illustrates a graph 502 that shows capacitance sensed by an NFC coil over time. Figure 5A illustrates that the capsule portion 202 of the wearable device is coupled to the cradle 200. When the capsule portion 202 of the wearable device is coupled to the cradle 200 and on the wrist of the user, a low level of capacitance is detected by an NFC coil 504 of the wearable device (also referred to as metallic data-transmission component 900 in Figure 5A).

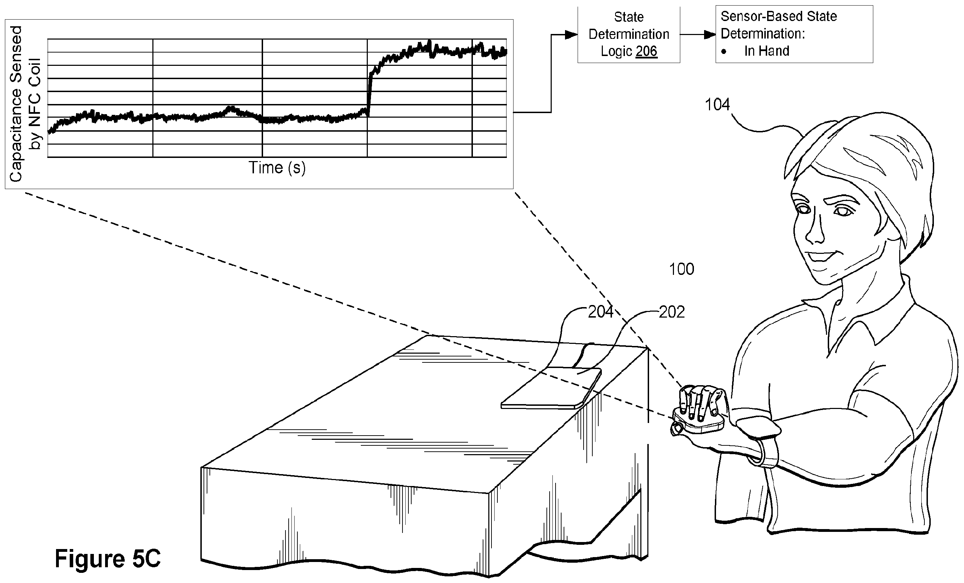

Figure 5C illustrates capsule portion 202 being detached from the cradle portion 200, and in the hand of the user 104. As shown in graph 502 of Figure 5C the sensed capacitance increases when the user holds the capsule portion 202 in their hand. This increased capacitance occurs as a result of the increased skin contact compared to wearing the capsule portion 202 on the user's wrist.

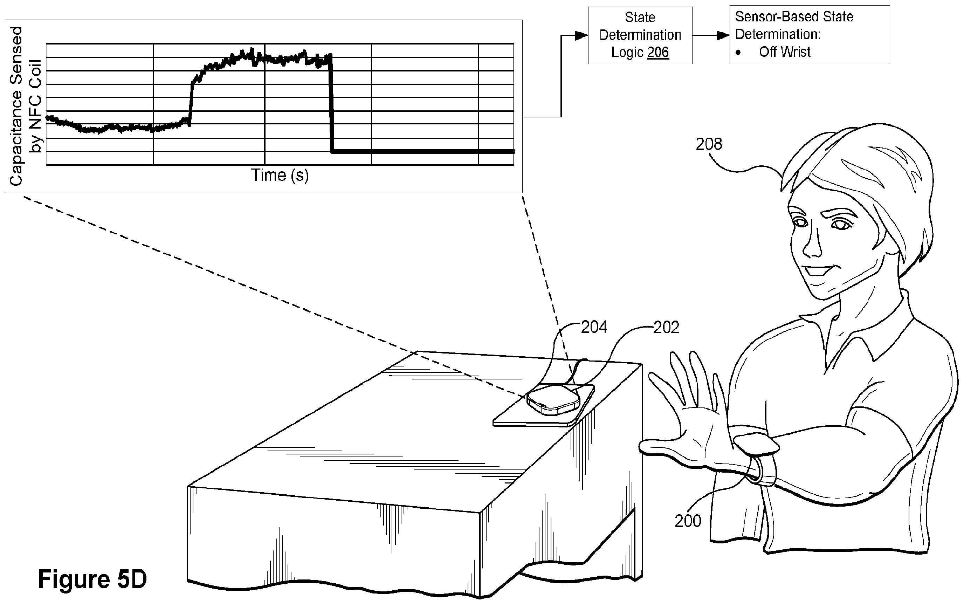

Figure 5D illustrates capsule portion 202 being placed on the charging pad 204 and no longer in contact with the user 104. As shown in graph 502 of Figure 5D the sensed capacitance decreases when the user is no longer holding the capsule portion 202 in their hand. This decreased capacitance occurs as a result of there being no skin contact compared wearing the capsule portion 202 on the wrist of the user 104 or being placed in a hand of a user 104.

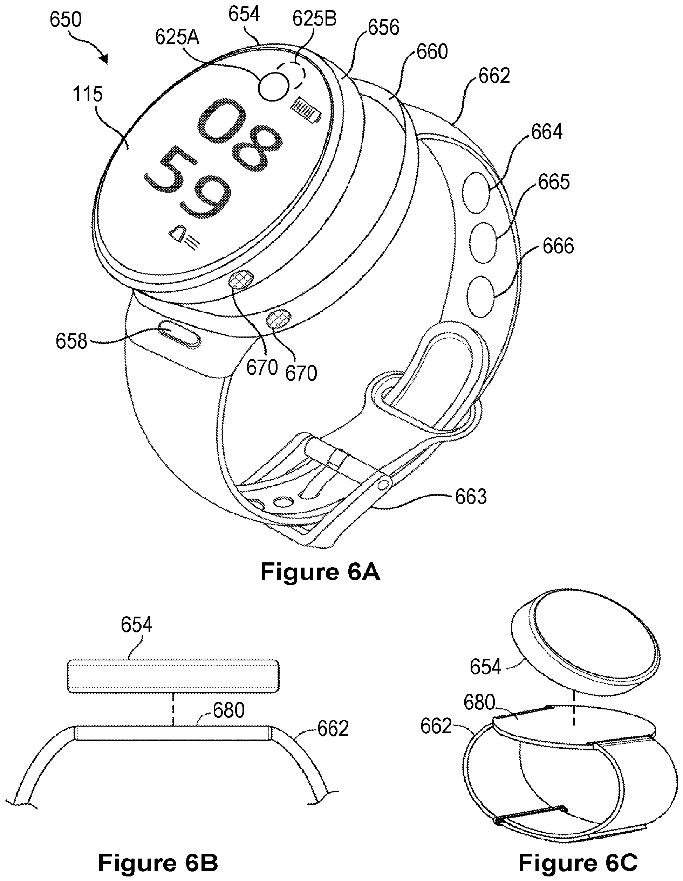

Figures 6A-6C illustrate an example wrist-wearable device 650, in accordance with some embodiments. The wearable device 100 shown and described in reference to Figures 1A-5C can be an instance of the wearable device 650 (also referred to as wrist-wearable device 650), including capsule portion 202 and cradle portion 200, such that capsule portion 202 and cradle portion 200 (also referred to as band portion 200) should be understood to have the features of the capsule portion 654 (also referred to as watch body 654) and band portion 662 (also referred to as watch body 662) of wearable device 650 and vice versa. Figure 6A illustrates a perspective view of the wrist-wearable device 650 that includes a capsule portion 654 decoupled from a band portion 662. Capsule portion 654 and band portion 662 can have a substantially rectangular or circular shape and can be configured to allow a user to wear the wrist-wearable device 650 on a body part (e.g., a wrist). The wrist-wearable device 650 can include a retaining mechanism 663 (e.g., a buckle, a hook and loop fastener, etc.) for securing band portion 662 to the user's wrist. The wrist-wearable device 650 can also include a coupling mechanism 660 (e.g., a cradle) for detachably coupling the capsule portion 654 (via a coupling surface 656 of the watch body 654) to band portion 662.

Functions executed by the wrist-wearable device 650 can include, without limitation, display of visual content to the user (e.g., visual content displayed on display screen 115), sensing user input (e.g., sensing a touch on button 658, sensing biometric data on sensor 664, sensing neuromuscular signals on neuromuscular sensor 665, etc.), messaging (e.g., text, speech, video, etc.), image capture, wireless communications (e.g., cellular, near field, WiFi, personal area network, etc.), location determination, financial transactions, providing haptic feedback, alarms, notifications, biometric authentication, health monitoring, sleep monitoring, etc. The wrist-wearable device 650 is configured to perform, without limitation, the functions described above in reference to Figures 1A-5C, such that the wrist-wearable device is able to make determinations about whether to make modifications to operating characteristics based on sensor data from at least two different sensors (as described above in reference to Figures 1A-5C) and can then, if it is determined that modifications to operating characteristics are needed, proceed to modify the operating characteristics based on the approaches and techniques. These functions can be executed independently in watch body 654, independently in watch band 662, and/or in communication between watch body 654 and watch band 662. In some embodiments, functions can be executed on the wrist-wearable device 650 in conjunction with an artificial-reality environment which includes, but is not limited to, virtual-reality (VR) environments (including non-immersive, semi-immersive, and fully-immersive VR environments), augmented-reality environments (including marker-based augmented-reality environments, markerless augmented-reality environments, location-based augmented-reality environments, and projection-based augmented-reality environments), hybrid reality, and other types of mixed-reality environments. As the skilled artisan will appreciate upon reading the descriptions provided herein, the novel wearable devices described herein can be used with any of these types of artificial-reality environments.

The watch band 662 can be configured to be worn by a user such that an inner surface of the watch band 662 is in contact with the user's skin. When worn by a user, sensor 664 is in contact with the user's skin. The sensor 664 can be a biosensor that senses a user's heart rate, saturated oxygen level, temperature, sweat level, muscle intentions, or a combination thereof (any of these can be examples of the biometric sensor described above and used in conjunction with the positional-state determinations described herein, and can also be associated with the capsule portion instead of the band portion). The watch band 662 can include multiple sensors 664 that can be distributed on an inside and/or an outside surface of the watch band 662. Additionally, or alternatively, the watch body 654 can include the same or different sensors than the watch band 662 (or the watch band 662 can include no sensors at all in some embodiments). For example, multiple sensors can be distributed on an inside and/or an outside surface of watch body 654. The watch body 654 (e.g., a capsule portion) can include, without limitation, a magnetic field sensor 120 (as shown in Figure 1A), antenna return loss sensor 124, front-facing image sensor 625A and/or rear-facing image sensor 625B, a biometric sensor (e.g., biometric sensor 126, as shown in Figure 1A), an IMU, a heart rate sensor, a saturated oxygen sensor, a neuromuscular sensor(s), an altimeter sensor, a temperature sensor, a bioimpedance sensor, a pedometer sensor, an optical sensor, a touch sensor (e.g., capacitive sensor 122 shown in figure 1A), a sweat sensor, a sensor or component configured to detect capacitance, etc. The sensor 664 can also include a sensor that provides data about a user's environment including a user's motion (e.g., an IMU), altitude, location, orientation, gait, or a combination thereof. The sensor 664 can also include a light sensor (e.g., an infrared light sensor, a visible light sensor) that is configured to track a position and/or motion of watch body 654 and/or watch band 662. Watch band 662 can transmit the data acquired by the sensor 664 to watch body 654 using a wired communication method (e.g., a UART, a USB transceiver, etc.) and/or a wireless communication method (e.g., near field communication, Bluetooth TM, etc.). Watch band 662 can be configured to operate (e.g., to collect data using sensor 664) independent of whether watch body 654 is coupled to or decoupled from watch band 662.

The watch band 662 and/or watch body 654 can include a haptic device 666 (e.g., a vibratory haptic actuator) that is configured to provide haptic feedback (e.g., a cutaneous and/or kinesthetic sensation, etc.) to the user's skin. The sensor 664 and/or haptic device 666 can be configured to operate in conjunction with multiple applications including, without limitation, health monitoring, social media, game playing, and artificial reality (e.g., the applications associated with artificial reality).

In some examples, the watch band 662 can include a neuromuscular sensor 665 (e.g., an electromyography (EMG) sensor, a mechanomyogram (MMG) sensor, a sonomyography (SMG) sensor, etc.). Neuromuscular sensor 665 can sense a user's intention to perform certain motor actions (this sensor 665 can be another example of a sensor used as the biometric sensor in conjunction with the positional-state determinations described herein). The sensed muscle intention can be used to control certain user interfaces displayed on the display 115 of the device 102 and/or can be transmitted to device responsible for rendering an artificial-reality environment (e.g., a head-mounted display) to perform an action in an associated artificial-reality environment, such as to control the motion of a virtual device displayed to the user.

Signals from neuromuscular sensor 665 can be used to provide a user with an enhanced interaction with a physical object and/or a virtual object in an artificial-reality application generated by an artificial-reality system (e.g., user interface objects presented on the display 115, or another computing device (e.g., a head-mounted display)). Signals from neuromuscular sensor 665 can be obtained (e.g., sensed and recorded) by one or more neuromuscular sensors 665 of watch band 662. Although Figure 6A shows one neuromuscular sensor 665, watch band 662 can include a plurality of neuromuscular sensors 665 arranged circumferentially on an inside surface of watch band 662 such that the plurality of neuromuscular sensors 665 contact the skin of the user. Watch band 662 can include a plurality of neuromuscular sensors 665 arranged circumferentially on an inside surface of watch band 662. Neuromuscular sensor 665 can sense and record neuromuscular signals from the user as the user performs muscular activations (e.g., movements, gestures, etc.). The muscular activations performed by the user can include static gestures, such as placing the user's hand palm down on a table; dynamic gestures, such as grasping a physical or virtual object; and covert gestures that are imperceptible to another person, such as slightly tensing a joint by co-contracting opposing muscles or using sub-muscular activations. The muscular activations performed by the user can include symbolic gestures (e.g., gestures mapped to other gestures, interactions, or commands, for example, based on a gesture vocabulary that specifies the mapping of gestures to commands).

The wrist-wearable device 650 can include a coupling mechanism (also referred to as a cradle) for detachably coupling watch body 654 to watch band 662. A user can detach watch body 654 from watch band 662 to reduce the encumbrance of the wrist-wearable device 650 to the user. The wrist-wearable device 650 can include a coupling surface 656 on the watch body 654 and/or coupling mechanism(s) 660 (e.g., a cradle, a tracker band, a support base, a clasp). A user can perform any type of motion to couple watch body 654 to watch band 662 and to decouple watch body 654 from watch band 662. For example, a user can twist, slide, turn, push, pull, or rotate watch body 654 relative to watch band 662, or a combination thereof, to attach watch body 654 to watch band 662 and to detach watch body 654 from watch band 662.

As shown in the example of Figure 6A, watch band coupling mechanism 660 can include a type of frame or shell that allows watch body 654 coupling surface 656 to be retained within watch band coupling mechanism 660. Watch body 654 can be detachably coupled to watch band 662 through a friction fit, magnetic coupling, a rotation-based connector, a shear-pin coupler, a retention spring, one or more magnets, a clip, a pin shaft, a hook and loop fastener, or a combination thereof. In some examples, watch body 654 can be decoupled from watch band 662 by actuation of release mechanism 670. The release mechanism 670 can include, without limitation, a button, a knob, a plunger, a handle, a lever, a fastener, a clasp, a dial, a latch, or a combination thereof.

The wrist-wearable device 650 can include a single release mechanism 670 or multiple release mechanisms 670 (e.g., two release mechanisms 670 positioned on opposing sides of the wrist-wearable device 650 such as springloaded buttons). As shown in Figure 6A, the release mechanism 670 can be positioned on watch body 654 and/or watch band coupling mechanism 660. Although Figure 6A shows release mechanism 670 positioned at a corner of watch body 654 and at a corner of watch band coupling mechanism 660, the release mechanism 670 can be positioned anywhere on watch body 654 and/or watch band coupling mechanism 660 that is convenient for a user of wrist-wearable device 650 to actuate. A user of the wrist-wearable device 650 can actuate the release mechanism 670 by pushing, turning, lifting, depressing, shifting, or performing other actions on the release mechanism 670. Actuation of the release mechanism 670 can release (e.g., decouple) the watch body 654 from the watch band coupling mechanism 660 and the watch band 662 allowing the user to use the watch body 654 independently from watch band 662. For example, decoupling the watch body 654 from the watch band 662 can allow the user to capture images using rear-facing image sensor 625B.

Figure 6B is a side view and Figure 6C is a perspective view of another example of the wrist-wearable device 650. The wrist-wearable devices 650 of Figures 6B and 6C can include a watch body interface 680 (another example of a cradle for the capsule portion of the device 102). The watch body 654 can be detachably coupled to the watch body interface 680. Watch body 654 can be detachably coupled to watch body interface 680 through a friction fit, magnetic coupling, a rotation-based connector, a shear-pin coupler, a retention spring, one or more magnets, a clip, a pin shaft, a hook and loop fastener, or a combination thereof.

In some examples, watch body 654 can be decoupled from watch body interface 680 by actuation of a release mechanism. The release mechanism can include, without limitation, a button, a knob, a plunger, a handle, a lever, a fastener, a clasp, a dial, a latch, or a combination thereof. In some examples, the wristband system functions can be executed independently in watch body 654, independently in watch body interface 680, and/or in communication between watch body 654 and watch body interface 680. Watch body interface 680 can be configured to operate independently (e.g., execute functions independently) from watch body 654. Additionally, or alternatively, watch body 654 can be configured to operate independently (e.g., execute functions independently) from watch body interface 680. Watch body interface 680 and/or watch body 654 can each include the independent resources required to independently execute functions. For example, watch body interface 680 and/or watch body 654 can each include a power source (e.g., a battery), a memory, data storage, a processor (e.g., a CPU), communications, a light source, and/or input/output devices.

In this example, watch body interface 680 can include all of the electronic components of watch band 662. In additional examples, one or more electronic components can be housed in watch body interface 680 and one or more other electronic components can be housed in portions of watch band 662 away from watch body interface 680.

Figure 7 is a block diagram of a wrist-wearable device system 700, according to at least one embodiment of the present disclosure. The device 100 described in detail above is an example wrist-wearable device system 700, so device 100 will be understood to include the components shown and described for system 700 below. The wrist-wearable device system 700 can have a split architecture (e.g., a split mechanical architecture, a split electrical architecture) between a watch body 704 (e.g., a capsule 654/ capsule portion 202) and a watch band 712 (e.g., a band portion 662/ cradle portion 200), which was described above in reference to Figures 6A-6C. Each of watch body 704 and watch band 712 can have a power source, a processor, a memory, sensors, a charging device, and a communications device that enables each of watch body 704 and watch band 712 to execute computing, controlling, communication, and sensing functions independently in watch body 704, independently in watch band 712, and/or in communication between watch body 704 and watch band 712.

For example, watch body 704 can include capacitive sensor 122 (or a device such as an NFC sensor 715 that can in some embodiments act as a capacitive sensor), magnetic field sensor 120, antenna return-loss (RL) sensor 124, biometric sensor 126, battery 728 , CPU 726, storage 702, heart rate sensor 758, EMG sensor 746, SpO₂ sensor 754, altimeter 748, IMU 742, random access memory 703, charging input 730 and communication devices NFC 715, LTE 718, and WiFi/BLUETOOTH^{™} 720. Similarly, watch band 712 can include battery 738, microcontroller unit 752, memory 750, heart rate sensor 758, EMG sensor 746, SpO₂ sensor 754, altimeter 748, IMU 742, charging input 734 and wireless transceiver 740. Memory 750 (and/or storage 702) may further include device state table, an example of which is shown in Figure 1A-2G and 5A-5C. In some examples, a level of functionality of at least one of watch band 712 or watch body 704 can be modified when watch body 704 is detached from watch band 712. The level of functionality that can be modified can include the functionality of at least one sensor (e.g., heart rate sensor 758, EMG sensor 746, etc.). Each of watch body 704 and watch band 712 can execute instructions stored in storage 702 and memory 750 respectively that enables at least one sensor (e.g., heart rate sensor 758, EMG sensor 746, etc.) in watch band 712 to acquire data when watch band 712 is detached from watch body 704 and when watch band 712 is attached to watch body 704.

Watch body 704 and watch band 712 can further execute instructions stored in storage 702 and memory 750 respectively that enables watch band 712 to transmit the acquired data to watch body 704 (or other computing device such as a head mounted display or other computing device) using wired communications 727 and/or wireless transceiver 740. For example, watch body 704 can display visual content to a user on touchscreen display 713 (e.g., an instance of display 115) and play audio content on speaker 125. Watch body 704 can receive user inputs such as audio input from microphone 127 and touch input from buttons 724. Watch body 704 can also receive inputs associated with a user's location and/or surroundings. For example, watch body 704 can receive location information from GPS 716 and/or altimeter 748 of watch band 712.

Watch body 704 can receive image data from at least one image sensor 135 (e.g., a camera). Image sensor 135 can include front-facing image sensor 625A (Figure 6A) and/or rear-facing image sensor 625B (Figure 6B). Front-facing image sensor 625A and/or rear-facing image sensor 625B can capture wide-angle images of the area surrounding front-facing image sensor 625A and/or rear-facing image sensor 625B such as hemispherical images (e.g., at least hemispherical, substantially spherical, etc.), 180-degree images, 360-degree area images, panoramic images, ultra-wide area images, or a combination thereof. In some examples, front-facing image sensor 625A and/or rear-facing image sensor 625B can be configured to capture images having a range between 45 degrees and 360 degrees. Certain input information received by watch body 704 (e.g., user inputs, etc.) can be communicated to watch band 712. Similarly, certain input information (e.g., acquired sensor data, neuromuscular sensor data, etc.) received by watch band 712 can be communicated to watch body 704.

Watch body 704 and watch band 712 can receive a charge using a variety of techniques. In some embodiments, watch body 704 and watch band 712 can use a wired charging assembly (e.g., power cords) to receive the charge. Alternatively, or in addition, watch body 704 and/or watch band 712 can be configured for wireless charging. For example, a portable charging device can be designed to mate with a portion of watch body 704 and/or watch band 712 and wirelessly deliver usable power to a battery of watch body 704 and/or watch band 712.

Watch body 704 and watch band 712 can have independent power and charging sources to enable each to operate independently. Watch body 704 and watch band 712 can also share power (e.g., one can charge the other) via power management IC 732 in watch body 704 and power management IC 736 in watch band 712. Power management IC 732 and power management IC 736 can share power over power and ground conductors and/or over wireless charging antennas.

Wrist-wearable device system 700 can operate in conjunction with a health monitoring application that acquires biometric and activity information associated with the user. The health monitoring application can be designed to provide information to a user that is related to the user's health. For example, wrist-wearable device system 700 can monitor a user's physical activity by acquiring data from IMU 742 while simultaneously monitoring the user's heart rate via heart rate sensor 758 and saturated blood oxygen levels via SpO₂ sensor 754. CPU 726 can process the acquired data and display health related information to the user on touchscreen display 713.

Wrist-wearable device system 700 can detect when watch body 704 and watch band 712 are connected to one another (e.g., mechanically connected and/or electrically or magnetically connected) or detached from one another. For example, pin(s), power/ground connections 760, wireless transceiver 740, and/or wired communications 727, can detect whether watch body 704 and watch band 712 are mechanically and/or electrically or magnetically connected to one another (e.g., detecting a disconnect between the one or more electrical contacts of power/ground connections 760 and/or wired communications 727). In some examples, when watch body 704 and watch band 712 are mechanically and/or electrically disconnected from one another (e.g., watch body 712 has been detached from watch band 712 as described with reference to Figures 6A-6C), watch body 704 and/or watch band 712 can operate with modified level of functionality (e.g., reduced functionality) as compared to when watch body 704 and watch band 712 are mechanically and/or electrically connected to one another. The modified level of functionality (e.g., switching from full functionality to reduced functionality and from reduced functionality to full functionality) can occur automatically (e.g., without user intervention) when wrist-wearable device system 700 determines that watch body 704 and watch band 712 are mechanically and/or electrically disconnected from one another and connected to each other, respectively.

Modifying the level of functionality (e.g., reducing the functionality in watch body 704 and/or watch band 712) can reduce power consumption in battery 728 and/or battery 738. For example, any of the sensors (e.g., heart rate sensor 758, EMG sensor 746, SpO₂ sensor 754, altimeter 748, etc.), processors (e.g., CPU 726, microcontroller unit 752, etc.), communications elements (e.g., NFC 715, GPS 716, LTE 718, WiFi/BLUETOOTH^{™} 720, etc.), or actuators (e.g., haptics 722, 749, etc.) can reduce functionality and/or power consumption (e.g., enter a sleep mode) when watch body 704 and watch band 712 are mechanically and/or electrically disconnected from one another. Watch body 704 and watch band 712 can return to full functionality when watch body 704 and watch band 712 are mechanically and/or electrically connected to one another. The level of functionality of each of the sensors, processors, actuators, and memory can be independently controlled.

As described above, wrist-wearable device system 700 can detect when watch body 704 and watch band 712 are coupled to one another (e.g., mechanically connected and/or electrically connected) or decoupled from one another. In some examples, watch body 704 can modify a level of functionality (e.g., activate and/or deactivate certain functions) based on whether watch body 704 is coupled to watch band 712. For example, CPU 726 can execute instructions that detect when watch body 704 and watch band 712 are coupled to one another and activate front-facing image sensor 625A. CPU 726 can activate front-facing image sensor 625A based on receiving user input (e.g., a user touch input from touchscreen display 713, a user voice command from microphone 127, a user gesture recognition input from EMG sensor 746, etc.).

When CPU 726 detects that watch body 704 and watch band 712 are decoupled from one another, CPU 726 can modify a level of functionality (e.g., activate and/or deactivate additional functions). For example, CPU 726 can detect when watch body 704 and watch band 712 are decoupled from one another and activate rear-facing image sensor 625B. CPU 726 can activate rear-facing image sensor 625B automatically (e.g., without user input) and/or based on receiving user input (e.g., a touch input, a voice input, an intention detection, etc.). Automatically activating rear-facing image sensor 625B can allow a user to take wide-angle images without having to provide user input to activate rear-facing image sensor 625B.

In some examples, rear-facing image can be activated based on an image capture criterion (e.g., an image quality, an image resolution, etc.). For example, rear-facing image sensor 625B can receive an image (e.g., a test image). CPU 726 and/or rear-facing image sensor 625B can analyze the received test image data and determine whether the test image data satisfies the image capture criterion (e.g., the image quality exceeds a threshold, the image resolution exceeds a threshold, etc.). Rear-facing image sensor 625B can be activated when the test image data satisfies the image capture criterion. Additionally, or alternatively, rear-facing image sensor 625B can be deactivated when the test image data fails to satisfy the image capture criterion.

In some examples, CPU 726 can detect when watch body 704 is coupled to watch band 1012 and deactivate rear-facing image sensor 625B. CPU 726 can deactivate rear-facing image sensor 625B automatically (e.g., without user input) and/or based on receiving user input (e.g., a touch input, a voice input, an intention detection, etc.). Deactivating rear-facing image sensor 625B can automatically (e.g., without user input) reduce the power consumption of watch body 704 and increase the battery charge time in watch body 704. In some examples, wrist-wearable device system 700 can include a coupling sensor 707 that senses whether watch body 704 is coupled to or decoupled from watch band 712. Coupling sensor 707 can be included in any of watch body 704, watch band 712, or watch band coupling mechanism 660 of Figures 6A-6C. Coupling sensor 707 (e.g., a proximity sensor) can include, without limitation, an inductive proximity sensor, a limit switch, an optical proximity sensor, a capacitive proximity sensor, a magnetic proximity sensor, an ultrasonic proximity sensor, or a combination thereof. CPU 726 can detect when watch body 704 is coupled to watch band 712 or decoupled from watch band 712 by reading the status of coupling sensor 707.

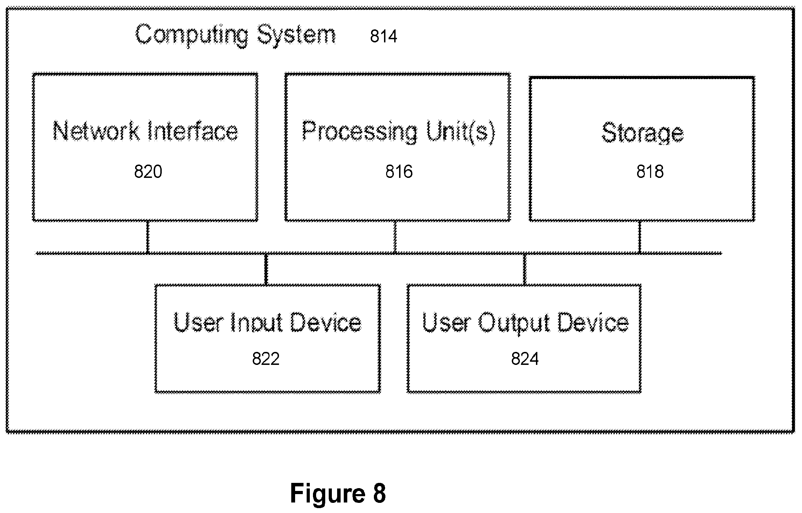

Various operations described herein can be implemented on computer systems. Figure 8 shows a block diagram of a representative computing system 814 usable to implement the present disclosure. In some embodiments, the wearable device 100 are implemented by the computing system 814. Computing system 814 can be implemented, for example, as a consumer device such as a smartphone, other mobile phone, tablet computer, wearable computing device (e.g., smart watch, eyeglasses, head wearable display), desktop computer, laptop computer, or implemented with distributed computing devices. The computing system 814 can be implemented to provide VR, AR, MR experience. In some embodiments, the computing system 814 can include conventional computer components such as processors 816, storage device 818, network interface 820, user input device 822, and user output device 824.

Network interface 820 can provide a connection to a wide area network (e.g., the Internet) to which WAN interface of a remote server system is also connected. Network interface 820 can include a wired interface (e.g., Ethernet) and/or a wireless interface implementing various RF data communication standards such as Wi-Fi, BLUETOOTH^{™}, or cellular data network standards (e.g., 3G, 4G, 5G, 60 GHz, LTE, etc.).

User input device 822 can include any device (or devices) via which a user can provide signals to computing system 814; computing system 814 can interpret the signals as indicative of particular user requests or information. User input device 822 can include any or all of a keyboard, touch pad, touchscreen, mouse or other pointing device, scroll wheel, click wheel, dial, button, switch, keypad, microphone, sensors (e.g., a motion sensor, an eye tracking sensor, etc.), and so on.

User output device 824 can include any device via which computing system 814 can provide information to a user. For example, user output device 824 can display images generated by or delivered to computing system 814 using a display. The display can incorporate various image generation technologies, e.g., a liquid crystal display (LCD), light-emitting diode (LED) including organic light-emitting diodes (OLED), projection system, cathode ray tube (CRT), or the like, together with supporting electronics (e.g., digital-to-analog or analog-to-digital converters, signal processors, or the like). A device such as a touchscreen that functions as both an input and output device can be used. Output device 824 can be provided in addition to or instead of a display. Examples include indicator lights, speakers, tactile "display" devices, printers, and so on.

Some implementations include electronic components, such as microprocessors, storage and memory that store computer program instructions in a computer readable storage medium (e.g., non-transitory computer readable medium). Many of the features described in this specification can be implemented as processes that are specified as a set of program instructions encoded on a computer readable storage medium. When these program instructions are executed by one or more processors, they cause the processors to perform various operations indicated in the program instructions. Examples of program instructions or computer code include machine code, such as is produced by a compiler, and files including higher-level codes that are executed by a computer, an electronic component, or a microprocessor using an interpreter. Through suitable programming, processor 816 can provide various functionalities for computing system 1814, including any of the functionalities described herein as being performed by a server or client, or other functionality associated with message management services.

It will be appreciated that computing system 814 is illustrative and that variations and modifications are possible. Computer systems used in connection with the present disclosure can have other capabilities not specifically described here. Further, while computing system 814 is described with reference to particular blocks, it is to be understood that these blocks are defined for convenience of description and are not intended to imply a particular physical arrangement of component parts. For instance, different blocks can be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Implementations of the present disclosure can be realized in a variety of apparatus including electronic devices implemented using any combination of circuitry and software.

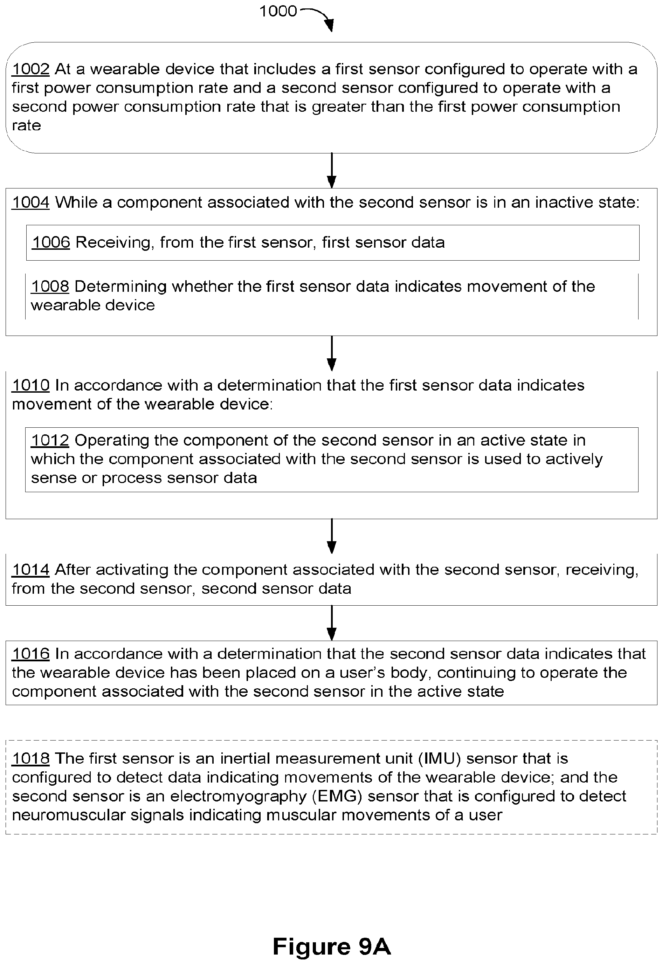

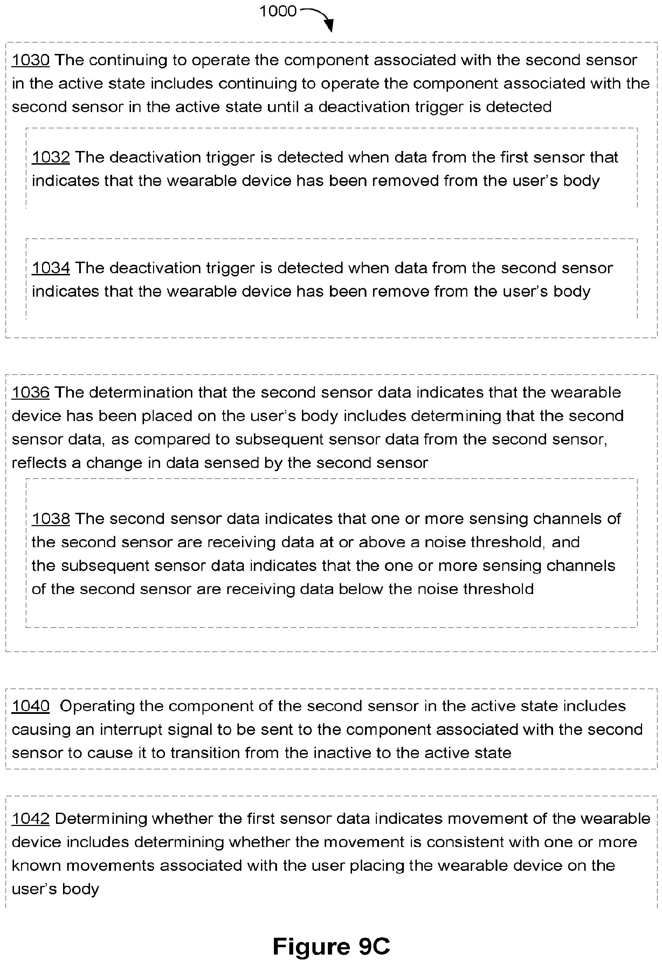

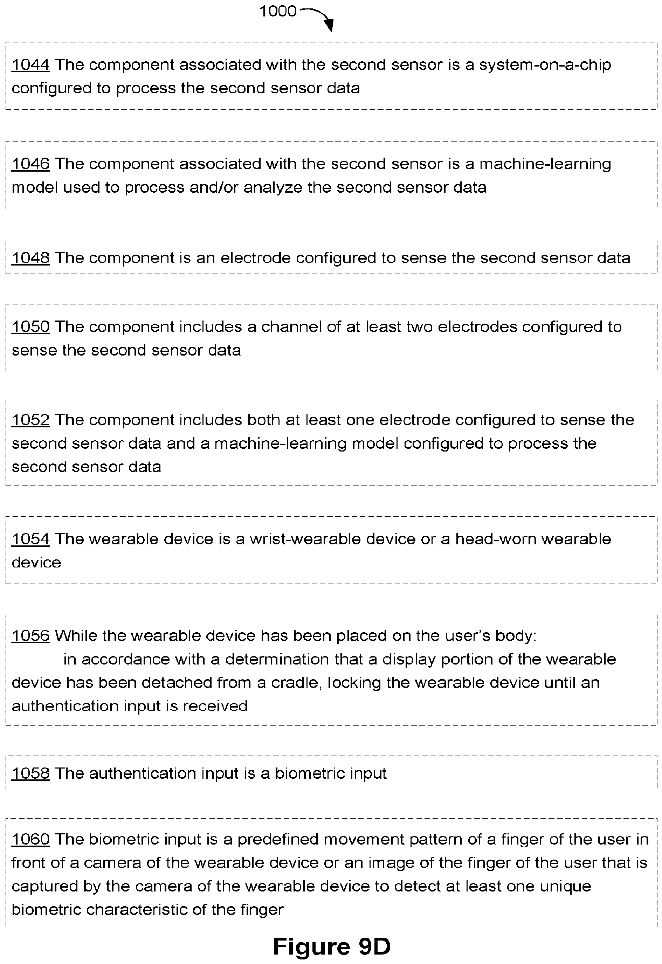

Figures 9A-9D illustrate an example flow chart of a method for activating and deactivating sensors of a wearable device, in accordance with some embodiments. The method 1000 is directed to optimizing power consumption in a wearable device. The method 1000 occurs at a wearable device that includes (1002) a first sensor configured to operate with a first power consumption rate and a second sensor configured to operate with a second power consumption rate that is greater than the first power consumption rate (e.g., Figures 1A-1E illustrated a wearable device 100 that has two sensors). The method includes, while a component associated with the second sensor is in an inactive state (1004) (e.g., EMG electrodes themselves, the circuitry used to process EMG signals (such as an analog front end), and/or a system-on-a-chip (SoC) for processing EMG signals can be in an inactive state (e.g., Figure 1A shows the second sensor in a powered off state, which is indicated by no data being recording in chart 108)): receiving (1006), from the first sensor, first sensor data, and determines (1008) whether the first sensor data indicates movement of the wearable device (e.g., Figures 1A-1E illustrate a state determination logic 110 box that indicates the components that are used for making a determination about the state of the device based on sensor data). The method further includes, in accordance with a determination (1010) that the first sensor data indicates movement of the wearable device (e.g., the determination can include checking that the first sensor data matches a predetermined pattern of movement that is associated with a user moving the wearable device to be worn or donned by the user on their forearm or wrist): operating (1012) the component of the second sensor in an active state in which the component associated with the second sensor is used to actively sense or process sensor data (e.g., the description associated with Figure 1B describes that the wearable device 100 watches the first sensor data before activating the second sensor). The method includes, after activating the component associated with the second sensor, receiving (1014), from the second sensor, second sensor data. The method includes, in accordance with a determination that the second sensor data indicates that the wearable device has been placed on a user's body, continuing (1016) to operate the component associated with the second sensor in the active state (e.g., Figure 1C illustrates wearable device 100 continues to keep the second sensor activated while it is determined the wearable device is on the wrist of the user).

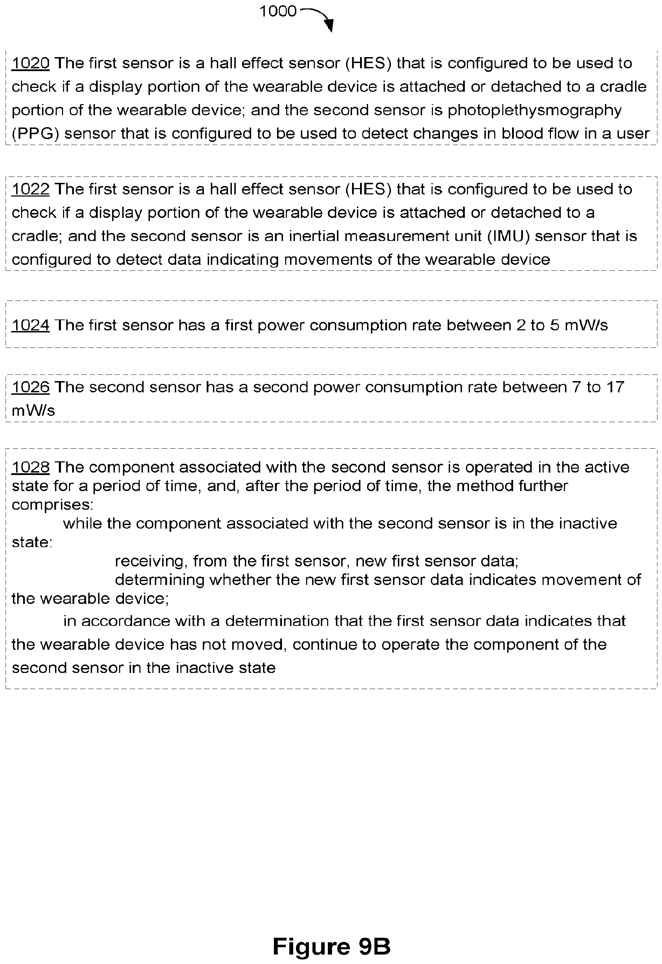

Operations 1018 and the other operations shown on Figures 9B-9D show various optional features that can be included at various points in conjunction with the operations of Figure 9A. For example, the first sensor can be selected from a group of many different sensors, and the second sensor can also be selected from a group of many different sensors.

In some embodiments, the first sensor is an inertial measurement unit (IMU) sensor that is configured to detect data indicating movements of the wearable device, and the second sensor is an electromyography (EMG) sensor that is configured to detect neuromuscular signals indicating muscular movements of a user (1018). In some embodiments, the first sensor is a different (i.e., not the same) sensor than the second sensor, and in some other embodiments the first sensor and second sensor are different components of the same sensor.

In some embodiments, the first sensor is a hall effect sensor (HES) that is configured to be used to check if a display portion of the wearable device is attached or detached to a cradle portion of the wearable device, and the second sensor is photoplethysmography (PPG) sensor that is configured to be used to detect changes in blood flow in a user (1020).

In some embodiments, the first sensor is a hall effect sensor (HES) that is configured to be used to check if a display portion of the wearable device is attached or detached to a cradle, and the second sensor is an inertial measurement unit (IMU) sensor that is configured to detect data indicating movements of the wearable device (1022).

In some embodiments, the first sensor has a first power consumption rate between 2 to 5 mW/s (milliwatts per second) (1024).

In some embodiments, the second sensor has a second power consumption rate between 7 to 17 mW/s (1026).

In some embodiments, the component associated with the second sensor is operated in the active state for a period of time, and, after the period of time, the method further comprises (1028): while the component associated with the second sensor is in the inactive state: receiving, from the first sensor, new first sensor data; and the wearable device determines whether the new first sensor data indicates movement of the wearable device. In some embodiments, the method includes in accordance with a determination that the first sensor data indicates that the wearable device has not moved, continuing to operate the component of the second sensor in the inactive state (e.g., Figure 1E illustrates the wearable device is off the wrist of the user and is stationary, and the wearable device 100 being switched to a low power mode while the data from the first sensor indicates there is no movement).

In some embodiments, the continuing to operate the component associated with the second sensor in the active state includes continuing (1030) to operate the component associated with the second sensor in the active state until a deactivation trigger is detected.

In some embodiments, the deactivation trigger is detected when data from the first sensor that indicates that the wearable device has been removed from the user's body (1032) (e.g., Figure 1E illustrates the wearable device is off the wrist of the user and is stationary, and the wearable device 100 being switched to a low power mode while the data from the first sensor indicates there is no movement).

In some embodiments, the deactivation trigger is detected when data from the second sensor indicates that the wearable device has been remove from the user's body (1034) (e.g., Figure 1E illustrates the wearable device is off the wrist of the user and is stationary, and the wearable device 100 being switched to a low power mode based on no data being received from the second sensor).

In some embodiments, the determination that the second sensor data indicates (1036) that the wearable device has been placed on the user's body includes determining that the second sensor data, as compared to subsequent sensor data from the second sensor, reflects a change in data sensed by the second sensor.

In some embodiments, the second sensor data indicates (1038) that one or more sensing channels of the second sensor are receiving data at or above a noise threshold (e.g., noise can include one or more of: inherent noise in the electronics equipment, ambient noise, motion artifact, inherent instability of signal, ECG artifacts, cross talk, electrode contact, transducer noise, or baseline shifts). In some embodiments, receiving data above a noise threshold includes identifying a pattern identified as not noise for a predetermined amount of time, and the subsequent sensor data indicates that the one or more sensing channels of the second sensor are receiving data below the noise threshold (e.g., one or more sensing channels reflect stable signals (while device is on wrist) as compared to noisy signals (while device is moving and being attached to wrist)). For example, Figure 2E illustrates a multichannel sensor, e.g., as indicated by the data in chart 214, there can be multiple sensors that have their data compiled together (e.g., multiple electrodes).

In some embodiments, operating (1040) the component of the second sensor in the active state includes causing an interrupt signal to be sent to the component associated with the second sensor to cause it to transition from the inactive to the active state.

In some embodiments, determining (1042) whether the first sensor data indicates movement of the wearable device includes determining whether the movement is consistent with one or more known movements associated with the user placing the wearable device on the user's body. For example, the description associated with Figure 1B discusses that the state determination logic 110 is configured to determine that the wearable device 100 is off the wrist of the user 104, and whether the wearable device 100 is moving. Figure 2D also shows in chart 212 that a sensor records a different pattern when the watch capsule 202 is on the wrist of the user 208 and/or connected to the watchband 200, than when the watch capsule 202 is not on the wrist of the user 208 and/or not connected to the watchband 200. In one example, the one or more known movements are detected based on a pattern of signals present in the first sensor data.

In some embodiments, the component associated with the second sensor is a system-on-a-chip configured to process the second sensor data (1044).

In some embodiments, the component associated with the second sensor is a machine-learning model used to process and/or analyze the second sensor data (1046) (e.g., the state determination logic 110 in Figures 1A-1E). In some embodiments, the second sensor can be configured to use multiple different types of machine-learning models, some of which can process many types of gestures and others of which can process a more limited set of gestures. In the case of the former type of machine-learning models, these can operate with a higher power-consumption level given the additional computational complexities and associated power requirements to operate a model that can detect many different types of gestures. So, in some embodiments, the lower-power-consumption model(s) can be always on to detect a limited set of gestures, but it is advantageous (for properly preserving limited power and computing resources, especially for wrist-wearable devices) to only active the higher-power-consumption model(s) when they are known to be needed (e.g., when a wrist-wearable device moves from an off-wrist state to a state in which data from the first sensor can be used to determine that the device is moving but that data cannot be used to determine at a high enough level of certainty (e.g., above a confidence level of 85% approximately) whether the device has been placed on a wrist or not). Thus, the first sensor data can be used as a trigger to figure out when to activate the power-hungry components of the second sensor, resulting in a system that efficiently makes use of limited computing and power resources.

In some embodiments, the component is an electrode configured to sense the second sensor data (1048). In some embodiments, the component includes a channel of at least two electrodes configured to sense the second sensor data (1050). For example, Figure 2E illustrates a multichannel sensor, e.g., as indicated by the data in chart 214, there can be multiple sensors that have their data compiled together (e.g., multiple electrodes).

In some embodiments, the component includes both at least one electrode configured to sense the second sensor data and a machine-learning model configured to process the second sensor data (1052). For example, this is similar to the different power-consumption levels for different types of machine-learning models, the underlying components used to sense the signals themselves (as compared to processing the signals after they are sensed) can also be activated at appropriate times to make efficient use of limited power and computing resources. These include individual electrodes, as well as groups of multiple electrodes (e.g., pairs) operating as sensor channels), such that individual sensors or groups of sensors can be activated at different points in time in accordance with the movement determinations made using the first sensor data. For example, the system can operate with a predetermined number of groups of sensors (e.g., 4 or 6) in an always on/active state and can determine when to activate a remaining number of groups of sensors (e.g., a remaining 2-10 additional groups) for assisting with detection of a more complex set of gestures.

In some embodiments, the wearable device is a wrist-wearable device or a head-worn wearable device (1054).

In some embodiments, while the wearable device has been placed on the user's body: in accordance with a determination that a display portion of the wearable device has been detached from a cradle, locking (1056) the wearable device until an authentication input is received (e.g., a waving of a finger detected by the rear-facing camera, as shown in Figures 13B-13C causes the wearable device to unlock). In some embodiments, a prompt is displayed at the wearable device informing the user that an authentication is required to unlock the device (e.g., user interface 1302 shown in Figures 13A-13C). In some embodiments, the authentication input is a biometric input (1058). In some embodiments, the biometric input is a predefined movement pattern of a finger of the user in front of a camera of the wearable device or an image of the finger of the user that is captured by the camera of the wearable device to detect at least one unique biometric characteristic of the finger (1060) (e.g., as shown in Figures13B-13C).

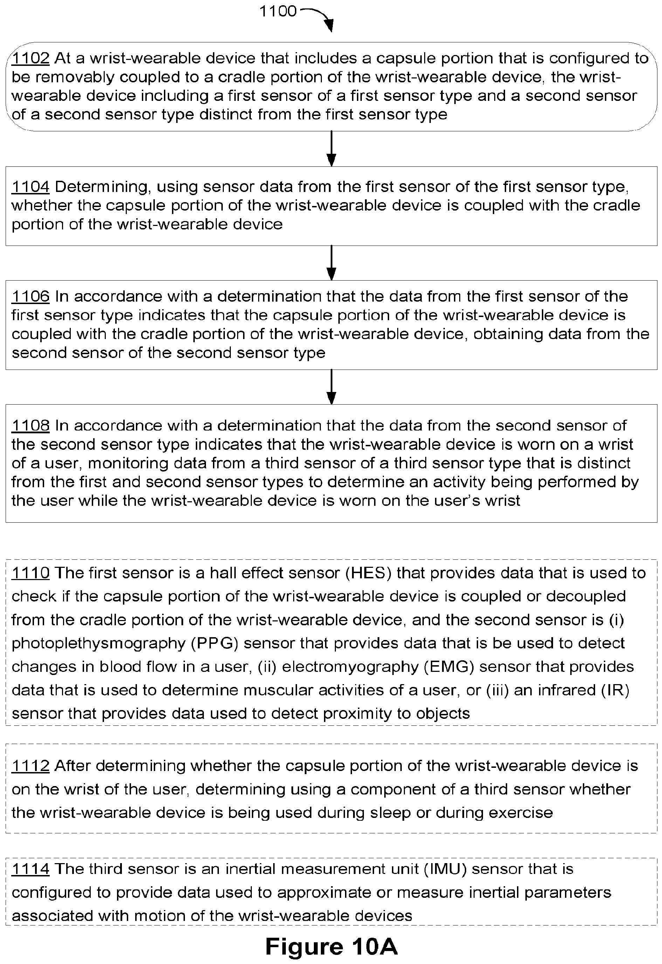

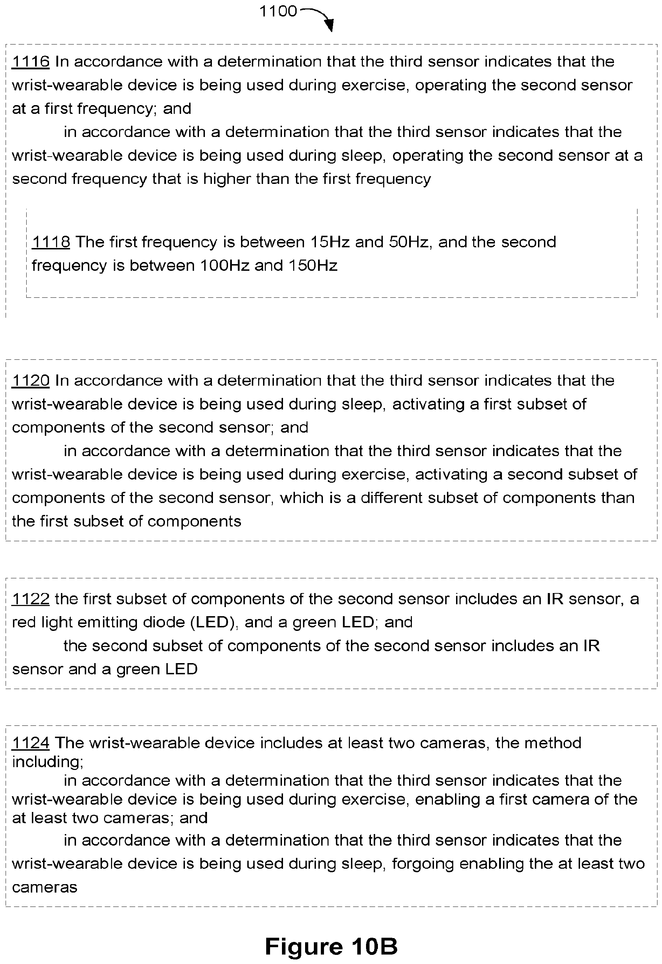

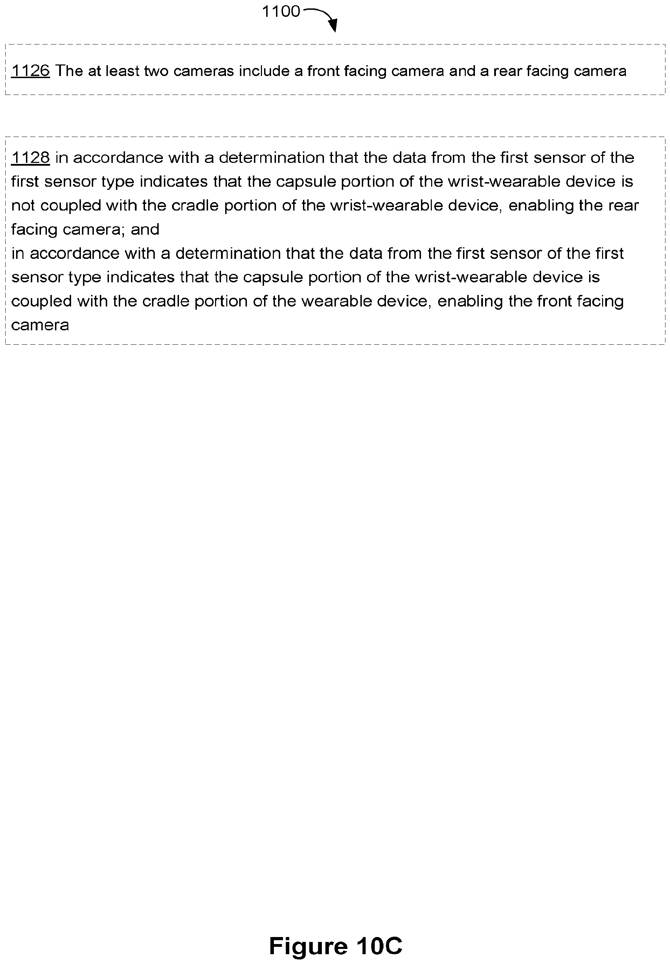

Figures 10A-10C illustrate an example flow chart of a method for activating and deactivating sensors of a wearable device that includes three different sensors, in accordance with some embodiments. The method 1100 is directed to optimizing power consumption in a wearable device. The method 1100 occurs at a wrist-wearable device that includes a capsule portion that is configured to be removably coupled to a cradle portion of the wrist-wearable device (in some embodiments, the capsule portion magnetically connects to the cradle portion), the wrist-wearable device including a first sensor of a first sensor type and a second sensor of a second sensor type distinct from the first sensor type (and the wrist-wearable device includes a third sensor of a third type distinct from the first sensor of the first type and the second sensor of the second type) (1102).

The method includes determining (1104) (e.g., at the capsule portion of the wrist-wearable device or a separate third connected device (e.g., a phone)), using sensor data from the first sensor of the first sensor type, whether the capsule portion of the wrist-wearable device is coupled with the cradle portion of the wrist-wearable device (e.g., Figure 2C illustrates that the first sensor, as illustrated by chart 210, is configured to provide data that the state determination logic 206 can use to determine if the watch capsule 202 has been placed on the cradle 200).

The method includes, in accordance with a determination that the data from the first sensor of the first sensor type indicates that the capsule portion of the wrist-wearable device is coupled with the cradle portion of the wrist-wearable device, (activating a second sensor from a powered-off state, and then) obtaining (1106) data from the second sensor of the second sensor type (e.g., Figure 2D shows the user 208 having connected the watch capsule 202 to the cradle 200, and the chart 212 indicating a pattern associated with capsule 202 to the cradle 200 being on the wrist of the user).

The method includes, in accordance with a determination that the data from the second sensor of the second sensor type indicates that the wrist-wearable device is worn on a wrist of a user, (activating a third sensor from a powered-off state, and then) monitoring (1108) data from a third sensor of a third sensor type that is distinct from the first and second sensor types to determine an activity being performed by the user while the wrist-wearable device is worn on the user's wrist (e.g., Figure 2D shows that in response to the state determination logic 206 determining that the watch is on the wrist of the user, the state determination logic 206 provides instructions for activating a third sensor, as indicated by the data in chart 214 being recorded).

In some embodiments, the first sensor of the first type is integrated into the capsule portion of the wrist-wearable device. In some embodiments, the first sensor of the first type is integrated into the cradle portion (including a watchband of the cradle portion) of the wrist-wearable device. In some embodiments, the second sensor of the second type is integrated into the capsule portion of the wrist-wearable device. In some embodiments, the second sensor of the second type is integrated into the cradle portion (including a watchband of the cradle portion) of the wrist-wearable device. In some embodiments, the third sensor of the third type is integrated into the capsule portion of the wrist-wearable device. In some embodiments, the third sensor of the third type is integrated into the cradle portion (including a watchband of the cradle portion) of the wrist-wearable device.

Operations 1110 and the other operations shown on Figures 10A-10C show various optional features that can be included at various points in conjunction with the operations of Figure 10A. For example, the first sensor can be selected from a group of many different sensors, the second sensor can also be selected from a group of many different sensors, and the third sensor can be selected from a group of many different sensors.

In some embodiments, the first sensor is a hall effect sensor (HES) that provides data that is used to check if the capsule portion of the wrist-wearable device is coupled or decoupled from the cradle portion of the wrist-wearable device, (e.g., the HES is used to sense the presence of the magnetic field of the cradle portion of the wearable device) and the second sensor is (i) photoplethysmography (PPG) sensor that provides data that is used to detect changes in blood flow in a user, (ii) electromyography (EMG) sensor that provides data that is used to determine muscular activities of a user, or (iii) an infrared (IR) sensor that provides data used to detect proximity to objects (1110).

In some embodiments, the method includes, after determining whether the capsule portion of the wrist-wearable device is on the wrist of the user, determining (1112) using a component of a third sensor whether the wrist-wearable device is being used during sleep or during exercise (e.g., Figure 2F illustrates that the state determination logic 206 has determined that the user has begun exercising, based at least on the data from the three sensors, and Figure 2G shows that the state determination logic 206 has determined that the user is sleeping, based on at least data recorded by the one or more sensors).

In some embodiments, the third sensor is an inertial measurement unit (IMU) sensor that is configured to provide data used to approximate or measure inertial parameters associated with motion of the wrist-wearable devices (1114) (e.g., Figure 2D shows that in response to the state determination logic 206 determining that the watch is on the wrist of the user, the state determination logic 206 provides instructions for activating a third sensor, which in some embodiments is an IMU sensor).

In some embodiments, in accordance with a determination that the third sensor indicates that the wrist-wearable device is being used during exercise, operating (1116) the second sensor at a first frequency (e.g., Figure 2F also shows that in accordance with a determination that the user is exercising the third sensor operates at a first frequency (e.g., 15Hz and 50Hz)), and in accordance with a determination that the third sensor indicates that the wrist-wearable device is being used during sleep, operating the second sensor at a second frequency (e.g., Figure 2G also shows that in accordance with a determination that the user is sleeping the third sensor operates at a second frequency that is higher than the first frequency (e.g., 100Hz and 150Hz)).

In some embodiments, the first frequency is between 15Hz and 50Hz (e.g., Figure 2F also shows that in accordance with a determination that the user is exercising the third sensor operates at a first frequency (e.g., 15Hz and 50Hz)), and the second frequency is between 100Hz and 150Hz (1118) (e.g., Figure 2G also shows that in accordance with a determination that the user is sleeping the third sensor operates at a second frequency (e.g., 100Hz and 150Hz)).

In some embodiments, the method includes, in accordance with a determination that the third sensor indicates that the wrist-wearable device is being used during sleep, activating (1120) a first subset of components of the second sensor (e.g., as shown in step 318 of Figure 3, when the IMU sensor provides data that indicates the user is sleeping (e.g., the data provided by the IMU corresponds to a predetermined pattern that indicates a user wearing the wearable device is sleeping), the rear-facing camera is disabled, the front-facing camera is disabled, the red LED, the green LED, and the IR on the AFE are enabled, and the sensors are configured to operate at 128Hz). In some embodiments, the method includes, in accordance with a determination that the third sensor indicates that the wrist-wearable device is being used during exercise, activating a second subset of components of the second sensor, which is a different subset of components than the first subset of components (e.g., as shown in step 320 of Figure 3, when the IMU sensor provides data that indicates that the user is exercising (e.g., the data provided by the IMU corresponds to a predetermined pattern that indicates a user wearing the wearable device is exercising), one or more sensors are operated at 25Hz, the green LED, and the IR on the AFE are enabled).

In some embodiments, the first subset of components of the second sensor includes an IR sensor, a red light emitting diode (LED), and a green LED (e.g., as shown in step 318 of Figure 3, when the IMU sensor provides data that indicates the user is sleeping (e.g., the data provided by the IMU corresponds to a predetermined pattern that indicates a user wearing the wearable device is sleeping), the rear-facing camera is disabled, the front-facing camera is disabled, the red LED, the green LED, and the IR on the AFE are enabled, and the sensors are configured to operate at 128Hz); and the second subset of components of the second sensor includes an IR sensor and a green LED (1122) (e.g., as shown in step 320 of Figure 3, when the IMU sensor provides data that indicates that the user is exercising (e.g., the data provided by the IMU corresponds to a predetermined pattern that indicates a user wearing the wearable device is exercising), one or more sensors are operated at 25Hz, the green LED, and the IR on the AFE are enabled).

In some embodiments, the wrist-wearable device includes at least two cameras (e.g., see Figure 3, illustrating when to activate and deactivate a front camera "Fcam" and a rear camera "Ream"). In some embodiments, the method includes, in accordance with a determination that the third sensor indicates that the wrist-wearable device is being used during exercise, enabling (1124) a first camera of the at least two cameras (e.g., Figure 3 illustrates in step 320 that the rear camera is off and the front camera is enabled); and in accordance with a determination that the third sensor indicates that the wrist-wearable device is being used during sleep, forgoing enabling the at least two cameras (e.g., Figure 3 illustrates in step 318 that the rear camera and front camera are off).

In some embodiments, the at least two cameras include a front facing camera and a rear facing camera (e.g., the front facing camera is on the same face as the display, and the rear-facing camera is on the opposite surface (1126) (e.g., the surface that mates to the cradle)).

In some embodiments, the method includes, in accordance with a determination that the data from the first sensor of the first sensor type indicates that the capsule portion of the wrist-wearable device is not coupled with the cradle portion of the wrist-wearable device, enabling (1128) the rear facing camera (e.g., step 304 of Figure 3 illustrates that the rear camera "Ream" is enabled and the front camera "Fcam" is not enabled when the capsule device is off the cradle), and in accordance with a determination that the data from the first sensor of the first sensor type indicates that the capsule portion of the wrist-wearable device is coupled with the cradle portion of the wearable device, enabling the front facing camera (e.g., step 306 of Figure 3 illustrates that the rear camera "Ream" is not enabled and the front camera "Fcam" is enabled when the capsule device is on the cradle). In some embodiments, the wearable device includes an ambient light sensor (e.g., the ambient light sensor is on the same face as the display). While the example in Figure 3 indicates that the ambient light sensor remains activated to improve PPG data (e.g., by filtering out ambient light noise), in some embodiments, the method includes, in accordance with a determination that the data from the first sensor of the first sensor type indicates that the capsule portion of the wrist-wearable device is coupled with the cradle portion of the wearable device, disabling the ambient light sensor.

Various examples discussed above related to use of data from a hall-effect sensor to make determinations as to whether or not a capsule is attached to a cradle portion on a band of a wearable device. As an alternative, or in addition, capacitively-sensed information about a user's digits relative to the capsule can also be used to assist with the cradle on/off determinations. In one example, a metallic data-transmission component (e.g., an NFC coil) can be repurposed to be used for this capacitive-sensing application. A skilled artisan would also appreciate that repurposing of the metallic data-transmission component can be used as a capacitive sensor independent of whether or not it is for the cradle on/off example application. More details are provided below. In some embodiments, the camera is on the opposite surface (1126) (e.g., the surface that mates to the cradle).

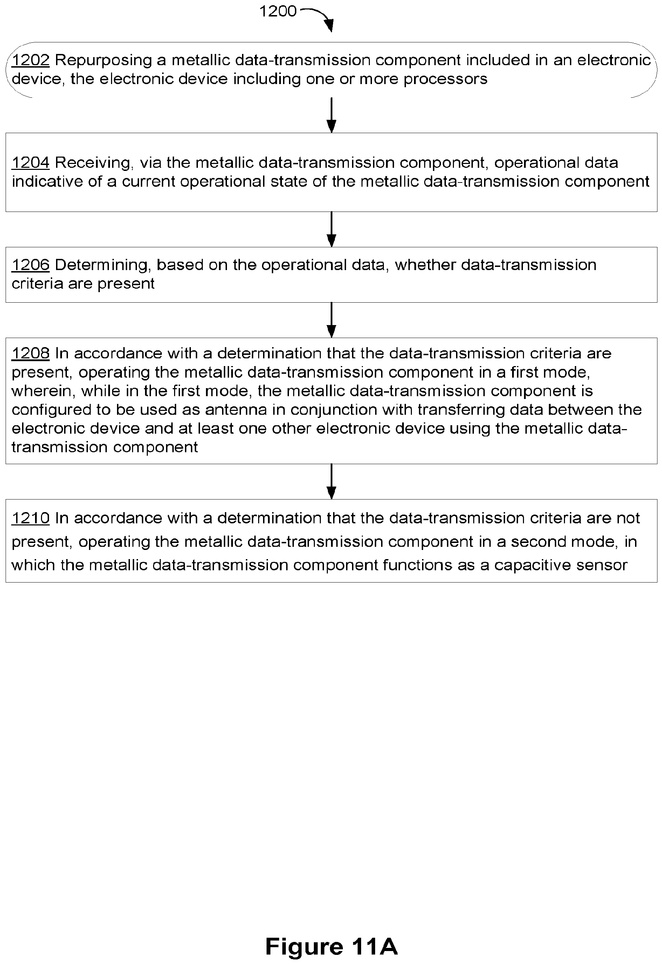

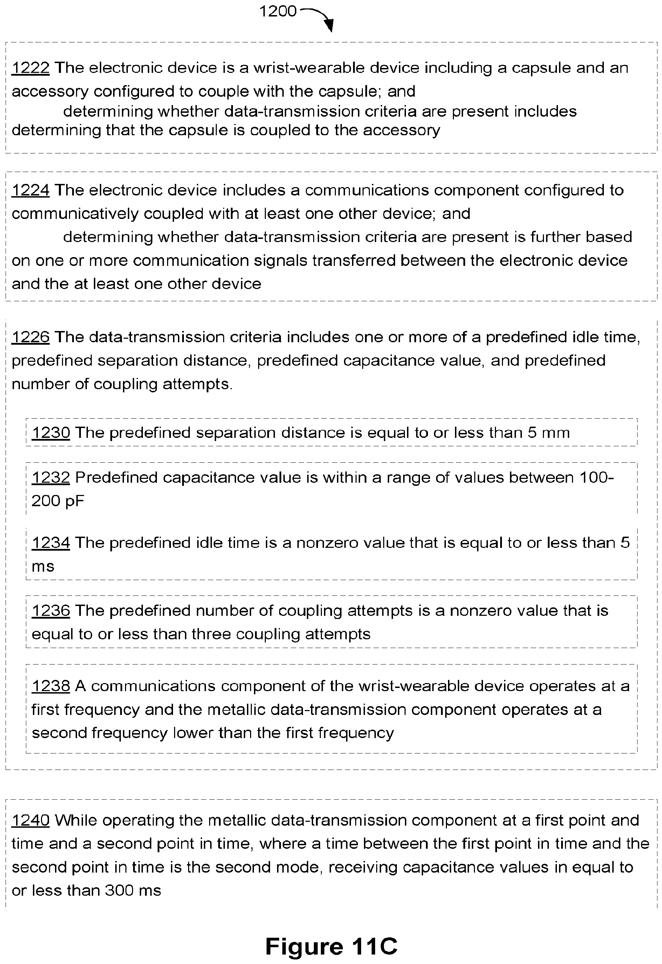

Figures 11A-11C illustrate an example flow chart of a method of using a metallic data-transmission component (e.g., an NFC coil) in a wearable device to also be used for this capacitive-sensing, in accordance with some embodiments. The method 1200 is directed to optimizing power consumption in a wearable device, and reduce the number of components in the device that use valuable resources (e.g., using a single component to do the job of another component). The method 1200 is a method of repurposing a metallic data-transmission component included in an electronic device, the electronic device including one or more processors (1202).

The method 1200 includes, receiving (1204), via the metallic data-transmission component, operational data indicative of a current operational state of the metallic data-transmission component. The method 1200 includes determining (1206), based on the operational data, whether data-transmission criteria are present.

The method 1200 also includes, in accordance with a determination that the data-transmission criteria are present, the electronic device operates (1208) the metallic data-transmission component in a first mode, wherein, while in the first mode, the metallic data-transmission component is configured to be used as antenna in conjunction with transferring data between the electronic device and at least one other electronic device using the metallic data-transmission component. In some embodiments, this first mode is a mode in which the metallic data-transmission component performs a first function and, as discussed below, when the metallic data-transmission component is operated in a second mode, it then performs a second function that is distinct from the first function.

The method 1200 includes that in accordance with a determination that the data-transmission criteria are not present, operating (1210) the metallic data-transmission component in a second mode, in which the metallic data-transmission component functions as a capacitive sensor.

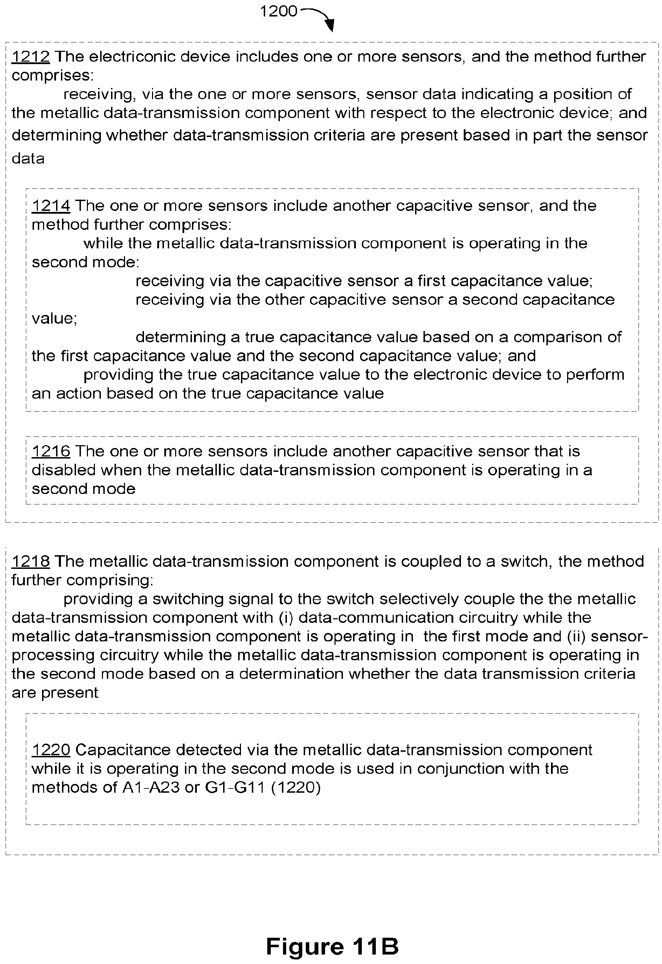

Operations 1212 and the other operations shown on Figures 11B-11C show various optional features that can be included at various points in conjunction with the operations of Figure 11A. For example, the data-transmission criteria includes one or more of a predefined idle time, predefined separation distance, predefined capacitance value, and predefined number of coupling attempts.

In some embodiments, the electronic device includes one or more sensors. In some embodiments, the method includes receiving (1212), via the one or more sensors, sensor data indicating a position of the metallic data-transmission component with respect to the electronic device; and determining whether data-transmission criteria are present based in part the sensor data.

In some embodiments, the one or more sensors include another capacitive sensor. In some embodiments, the method includes, while the metallic data-transmission component is operating in the second mode, receiving (1214), via the capacitive sensor, a first capacitance value. In some embodiments, the method includes, while the metallic data-transmission component is operating in the second mode, receiving, via the other capacitive sensor a second capacitance value. In some embodiments, the method includes, while the metallic data-transmission component is operating in the second mode, determining a true capacitance value based on a comparison of the first capacitance value and the second capacitance value. In some embodiments, the method includes, while the metallic data-transmission component is operating in the second mode, providing the true capacitance value to the electronic device to perform an action based on the true capacitance value (e.g., a touch screen input).

In some embodiments, the one or more sensors include another capacitive sensor that is disabled when the metallic data-transmission component is operating in a second mode (1216).

In some embodiments, the metallic data-transmission component is coupled to a switch. In some embodiments, the method includes, providing (1218) a switching signal to the switch to selectively couple the metallic data-transmission component with (i) data-communication circuitry while the metallic data-transmission component is operating in the first mode and (ii) sensor-processing circuitry while the metallic data-transmission component is operating in the second mode based on a determination whether the data-transmission criteria are present.

In some embodiments, capacitance detected via the metallic data-transmission component while it is operating in the second mode is used in conjunction with the methods of A1-A23 or G1-G11 (1220).

In some embodiments, the electronic device is a wrist-wearable device including a capsule and an accessory configured to couple with the capsule, and determining (1222) whether data-transmission criteria are present includes determining that the capsule is coupled to the accessory.

In some embodiments, the electronic device includes a communications component configured to communicatively coupled with at least one other device. In some embodiments, the communications components provides a beacon signal. In some embodiments, determining (1224) whether data-transmission criteria are present is further based on one or more communication signals transferred between the electronic device and the at least one other device. In some embodiments, a first signal transmitted by the metallic data-transmission component has a first wavelength and a second signal transmitted by the communications component has a second wavelength distinct from the first wavelength.

In some embodiments, the data-transmission criteria includes one or more of a predefined idle time, predefined separation distance, predefined capacitance value, and predefined number of coupling attempts (1226).

In some embodiments, the predefined separation distance is equal to or less than 5 millimeters (mm) (1230). In some embodiments, the metallic data-transmission component can detect capacitance up to a distance of at least 1 centimeter (cm), 2 cm, 3 cm.

In some embodiments, predefined capacitance value is within a range of values between 100-200 pF (1232).

In some embodiments, the predefined idle time is a nonzero value that is equal to or less than 5 ms seconds (1234).

In some embodiments, the predefined number of coupling attempts is a nonzero value that is equal to or less than three coupling attempts (1236).

In some embodiments, a wireless communications component of the wrist-wearable device (e.g., a BLUETOOTH^{™} radio) operates at a first frequency and the metallic data-transmission component operates at a second frequency lower than the first frequency (1238). In some embodiments, the metallic data-transmission component has an operations frequency of 13.5MHz. In some embodiments, due to the frequency differences there is no interferences.

In some embodiments, the method includes, while operating the metallic data-transmission component in the second mode, receiving capacitance values at a first point and time and a second point in time, where a time between the first point in time and the second point in time is less than 300 ms (1240). In some embodiments, the method includes, receiving capacitance values in less than 200 ms, and in some other embodiments less than 150ms. In some embodiments, preheating of a camera with SMA actuators takes about 300 to 500 ms, the metallic data-transmission component provides capacitance values quickly reducing the lag time between decoupling and activating the camera.

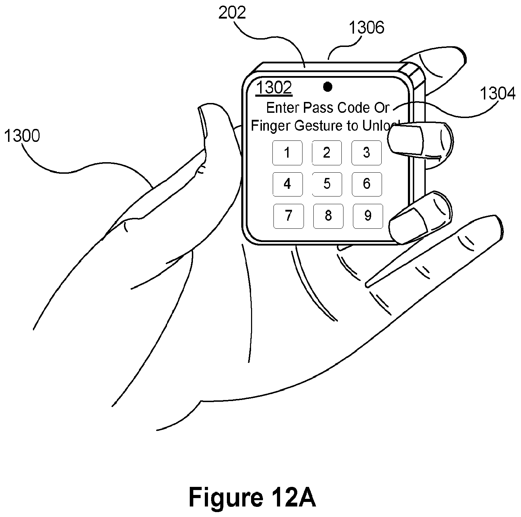

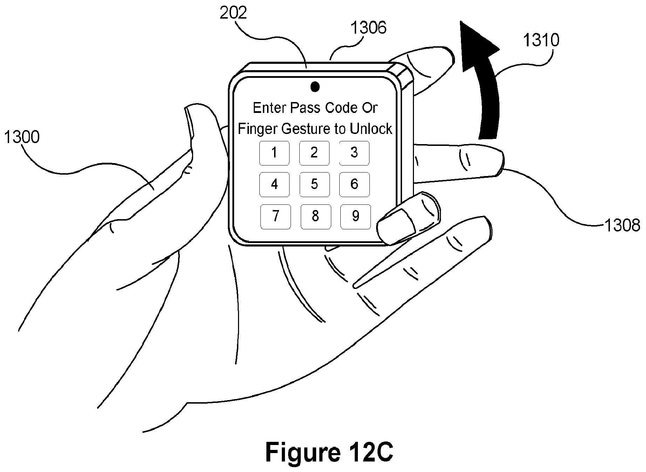

Figure 12A-12E illustrate a method of unlocking a wearable device using a gesture, in accordance with some embodiments. Figure 12A illustrates the capsule portion 202 being held in a hand 1300 of a user. When the capsule portion is removed from the cradle portion, or the capsule portion has made a determination that the device needs to be locked for other security reasons, a user interface 1302 is displayed notifying a user how to unlock the device. As shown, the user is prompted to either insert a pass code on a display 1304 of the capsule or provide a finger gesture. The finger gesture is detected by a rear facing camera 1306 (occluded), which can be configured to recognize a biometric input of a finger (e.g., a fingerprint, a motion of the finger, and/or vascular structure or another identifying feature).

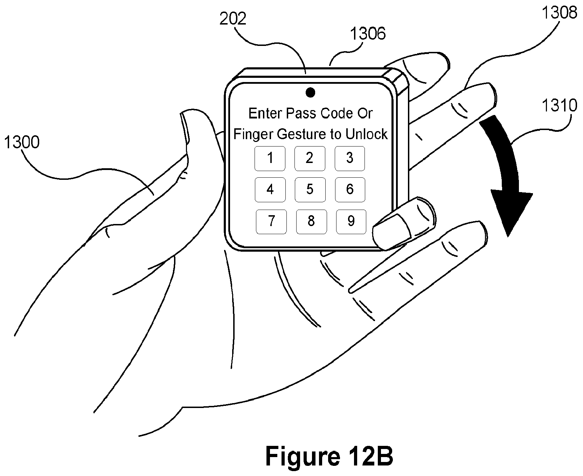

Figure 12B-12C illustrates a user of the device moving their middle finger 1308 in a waving manner 1310 to unlock the device using a gesture. While a waving gesture is shown, any number of other gestures can be used to unlock the device (e.g., curling a finger, articulating multiple fingers, etc.). In some embodiments, the rear facing or front-facing camera can be used to detect a gesture performed by the hand not holding the capsule 202.

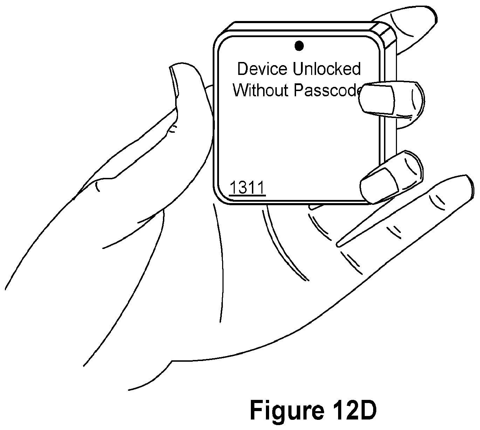

Figure 12D illustrates that in accordance with a determination that the gesture matches a gesture stored on the wearable device, the device is unlocked, as indicated by user interface 1311. In an unlocked state the user has access to all the files associated with the user who unlocked the device, and access to previously inaccessible features.

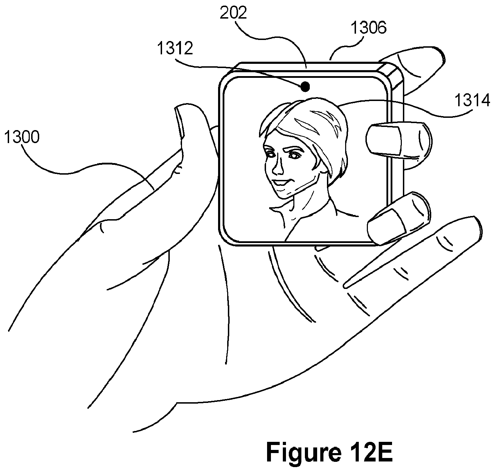

Figure 12E illustrates that in response to the device being unlocked, a front-facing camera 1312 is opened and a picture 1314 of the user is taken. In some embodiments, this picture 1314 is used for further verification that the user is the correct user unlocking the device (e.g., a two-step authentication).

It will be understood that, although the terms "first," "second," etc. may be used herein to describe various elements, these elements should not be limited by these terms. These terms are only used to distinguish one element from another.

The terminology used herein is for the purpose of describing particular embodiments only and is not intended to be limiting of the claims. As used in the description of the embodiments and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" can be construed to mean "when" or "upon" or "in response to determining" or "in accordance with a determination" or "in response to detecting," that a stated condition precedent is true, depending on the context. Similarly, the phrase "if it is determined [that a stated condition precedent is true]" or "if [a stated condition precedent is true]" or "when [a stated condition precedent is true]" can be construed to mean "upon determining" or "in response to determining" or "in accordance with a determination" or "upon detecting" or "in response to detecting" that the stated condition precedent is true, depending on the context.

The foregoing description, for purpose of explanation, has been described with reference to specific embodiments. However, the illustrative discussions above are not intended to be exhaustive or to limit the claims to the precise forms disclosed. Many modifications and variations are possible in view of the above teachings. The embodiments were chosen and described to best explain principles of operation and practical applications, to thereby enable others skilled in the art.

## Claims

1. A method of optimizing power consumption in a wearable device, the method comprising:
at a wearable device that includes a first sensor configured to operate with a first power consumption rate and a second sensor configured to operate with a second power consumption rate that is greater than the first power consumption rate:
while a component associated with the second sensor is in an inactive state:
receiving, from the first sensor, first sensor data;
determining whether the first sensor data indicates movement of the wearable device;
in accordance with a determination that the first sensor data indicates movement of the wearable device:
operating the component of the second sensor in an active state in which the component associated with the second sensor is used to actively sense or process sensor data;
after activating the component associated with the second sensor, receiving, from the second sensor, second sensor data; and
in accordance with a determination that the second sensor data indicates that the wearable device has been placed on a user's body, continuing to operate the component associated with the second sensor in the active state.

2. The method of claim 1, wherein:
the first sensor is an inertial measurement unit sensor that is configured to detect data indicating movements of the wearable device; and
the second sensor is an electromyography sensor that is configured to detect neuromuscular signals indicating muscular movements of a user.

3. The method of claim 1, wherein:
the first sensor is a Hall effect sensor that is configured to be used to check if a display portion of the wearable device is attached to or detached from a cradle portion of the wearable device; and
the second sensor is a photoplethysmography sensor that is configured to be used to detect changes in blood flow in a user.

4. The method of claim 1, wherein:
the first sensor is a Hall effect sensor that is configured to be used to check if a display portion of the wearable device is attached to or detached from a cradle; and
the second sensor is an inertial measurement unit sensor that is configured to detect data indicating movements of the wearable device.

5. The method of claim 1, wherein the first sensor has a first power consumption rate between 2 to 5 mW/s; and/or
wherein the second sensor has a second power consumption rate between 7 to 17 mW/s.

6. The method of claim 1, wherein the component associated with the second sensor is operated in the active state for a period of time, and, after the period of time, the method further comprises:
while the component associated with the second sensor is in the inactive state:
receiving, from the first sensor, new first sensor data;
determining whether the new first sensor data indicates movement of the wearable device;
in accordance with a determination that the first sensor data indicates that the wearable device has not moved, continuing to operate the component of the second sensor in the inactive state.

7. The method of claim 1, wherein the continuing to operate the component associated with the second sensor in the active state includes continuing to operate the component associated with the second sensor in the active state until a deactivation trigger is detected.

8. The method of claim 7, wherein the deactivation trigger is detected when data from the first sensor that indicates that the wearable device has been removed from the user's body; or
wherein the deactivation trigger is detected when data from the second sensor indicates that the wearable device has been removed from the user's body.

9. The method of claim 1, wherein the determination that the second sensor data indicates that the wearable device has been placed on the user's body includes determining that the second sensor data, as compared to subsequent sensor data from the second sensor, reflects a change in data sensed by the second sensor; and, optionally:
wherein:
the second sensor data indicates that one or more sensing channels of the second sensor are receiving data at or above a noise threshold; and
the subsequent sensor data indicates that the one or more sensing channels of the second sensor are receiving data below the noise threshold.

10. The method of claim 1, wherein operating the component of the second sensor in the active state includes causing an interrupt signal to be sent to the component associated with the second sensor to cause it to transition from the inactive to the active state; and/or
wherein determining whether the first sensor data indicates movement of the wearable device includes determining whether the movement is consistent with one or more known movements associated with the user placing the wearable device on the user's body.

11. The method of claim 1, wherein the wearable device is a wrist-wearable device or a head-worn wearable device.

12. The method of claim 1, comprising:
while the wearable device has been placed on the user's body:
in accordance with a determination that a display portion of the wearable device has been detached from a cradle, locking the wearable device until an authentication input is received.

13. The method of claim 12, wherein the authentication input is a biometric input; and
wherein the biometric input is a predefined movement pattern of a finger of the user in front of a camera of the wearable device or an image of the finger of the user that is capture by the camera of the wearable device to detect at least one unique biometric characteristic of the finger.

14. A wrist-wearable device that includes a first sensor configured to operate with a first power consumption rate and a second sensor configured to operate with a second power consumption rate that is greater than the first power consumption rate, and the wrist-wearable device is configured to perform or cause performance of:
while a component associated with the second sensor is in an inactive state:
receiving, from the first sensor, first sensor data;
determining whether the first sensor data indicates movement of the wearable device;
in accordance with a determination that the first sensor data indicates movement of the wearable device;
operating the component of the second sensor in an active state in which the component associated with the second sensor is used to actively sense or process sensor data;
after activating the component associated with the second sensor, receiving, from the second sensor, second sensor data; and
in accordance with a determination that the second sensor data indicates that the wearable device has been placed on a user's body, continuing to operate the component associated with the second sensor in the active state.

15. A non-transitory, computer-readable storage medium including instructions that, when executed by a wrist-wearable device that includes a first sensor configured to operate with a first power consumption rate and a second sensor configured to operate with a second power consumption rate that is greater than the first power consumption rate, cause the wrist-wearable device to:
while a component associated with the second sensor is in an inactive state:
receive, from the first sensor, first sensor data;
determine whether the first sensor data indicates movement of the wearable device;
in accordance with a determination that the first sensor data indicates movement of the wearable device:
operate the component of the second sensor in an active state in which the component associated with the second sensor is used to actively sense or process sensor data;
after activating the component associated with the second sensor, receive, from the second sensor, second sensor data; and
in accordance with a determination that the second sensor data indicates that the wearable device has been placed on a user's body, continue to operate the component associated with the second sensor in the active state.
